(19) Europäisches Patentamt
European Patent Office
Office européen des brevets

(11) EP 0 525 140 B1

(12) **EUROPÄISCHE PATENTSCHRIFT**

(45) Veröffentlichungstag und Bekanntmachung des Hinweises auf die Patenterteilung:
**19.04.2000 Patentblatt 2000/16**

(21) Anmeldenummer: **92903605.1**

(22) Anmeldetag: **07.02.1992**

(51) Int. Cl.[7]: **C09K 19/30**, C09K 19/12, C07C 43/225, C07C 43/174, C07C 43/192, C07C 25/18, C07C 25/24, C07C 39/42, C07C 63/00, C09K 19/14, C09K 19/16

(86) Internationale Anmeldenummer:
**PCT/EP92/00270**

(87) Internationale Veröffentlichungsnummer:
**WO 92/13928 (20.08.1992 Gazette 1992/22)**

(54) **FLÜSSIGKRISTALLINE VERBINDUNGEN**
LIQUID-CRYSTAL COMPOUNDS
COMPOSES DE CRISTAUX LIQUIDES

(84) Benannte Vertragsstaaten:
**CH DE ES FR GB IT LI NL**

(30) Priorität: **12.02.1991 DE 4104126**
**26.03.1991 DE 4109809**
**03.08.1991 DE 4125844**
**20.08.1991 DE 4127450**

(43) Veröffentlichungstag der Anmeldung:
**03.02.1993 Patentblatt 1993/05**

(73) Patentinhaber: **MERCK PATENT GmbH**
**64271 Darmstadt (DE)**

(72) Erfinder:
- **REIFFENRATH, Volker**
  **D-6101 Rossdorf (DE)**
- **PLACH, Herbert**
  **D-6100 Darmstadt (DE)**
- **PAULUTH, Detlef**
  **D-6100 Darmstadt (DE)**
- **HITTICH, Reinhard**
  **D-6101 Modautal 1 (DE)**
- **POETSCH, Eike**
  **D-6109 Mühltal 6 (DE)**
- **GEELHAAR, Thomas**
  **D-6500 Mainz (DE)**
- **WEBER, Georg**
  **D-6106 Erzhausen (DE)**
- **BARTMANN, Ekkehard**
  **D-6106 Erzhausen (DE)**

(56) Entgegenhaltungen:
**EP-A- 0 090 183**
**EP-A- 0 255 236**
**EP-A- 0 316 715**
**EP-A- 0 377 469**
**WO-A-90/01021**
**WO-A-90/01056**
**WO-A-90/13610**
**WO-A-91/03450**

- **Patent Abstracts of Japan, Band 9, Nr. 35 (C-265), 9. Februar 1985, & JP,A,59175454 (CHISSO) 4. Oktober 1984, siehe Zusammenfassung**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 525 140 B1

## Beschreibung

Die Erfindung betrifft flüssigkristalline Verbindungen der Formel I,

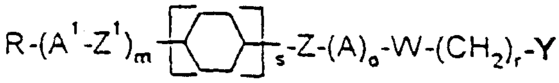

I

worin

- R: H, einen unsubstituierten, einen einfach durch CN oder $CF_3$ oder einen mindestens einfach durch Halogen substituierten Alkyl- oder Alkenylrest mit 1 bis 15 C-Atomen, wobei in diesen Resten auch eine oder mehrere $CH_2$-Gruppen jeweils unabhängig voneinander durch -O-,

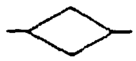,

-S-, -CO-, -CO-O-, -O-CO-oder -O-CO-O- so ersetzt sein können, daß O-Atome nicht direkt miteinander verknüpft sind,

- $Z^1$ und Z: jeweils unabhängig voneinander $-CH_2CH_2-$, -CH=CH-, -C≡C- oder eine Einfachbindung, einer der Reste $Z^1$ und Z auch $-(CH_2)_4-$ oder $-CH=CH-CH_2-CH_2-$,
- A: einen substituierten 1,4-Phenylenring, der in 2-, 2,3- oder 2,6-Stellung durch Fluor substituiert ist,
- und $A^1$: ein trans-1,4-Cyclohexylen, worin auch ein oder zwei nicht benachbarte $CH_2$-Gruppen durch -O- ersetzt sein können, oder unsubstituiertes oder ein- oder zweifach durch Fluor und/oder Cl-Atome substituiertes 1,4-Phenylen, worin auch eine oder zwei CH-Gruppen durch N ersetzt sein können,
- m: 0, 1, 2 oder 3,
- o: 1 oder 2,
- s: 0, 1 oder 2, wobei $(m + s + o) \geq 2$ ,
- W: -O- oder -COO-,
- r: 1 bis 7, und
- Y: F, Cl oder $CF_3$

bedeuten.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Die bisher für diesen Zweck eingesetzten Substanzen besitzen gewisse Nachteile, beispielsweise zu hohe Schmelzpunkte, zu niedrige Klärpunkte, zu geringe Stabilität gegenüber der Einwirkung von Wärme, Licht oder elektrischen Feldern, einen zu niedrigen elektrischen Widerstand, eine zu hohe Temperaturabhängigkeit der Schwellenspannung, ungünstige elastische und/oder dielektrische Eigenschaften.

Insbesondere bei Anzeigen von Supertwisttyp (STN) mit Verdrillungswinkeln von deutlich mehr als 220° oder bei Anzeigen mit aktiver Matrix weisen die bisher eingesetzten Materialien Nachteile auf.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie und eine hohe Nematogenität.

Es wurde nun gefunden, daß Verbindungen der Formel I als Komponenten flüssigkristalliner Medien vorzüglich geeignet sind. Insbesondere verfügen sie über vergleichsweise niedrige Viskositäten und hohe Nematogenitäten. Mit ihrer Hilfe lassen sich stabile flüssigkristalline Medien mit breitem Mesophasenbereich und vorteilhaften Werten für die optische und dielektrische Anisotropie erhalten. Diese Medien weisen ferner ein sehr gutes Tieftemperaturverhalten auf, d.h. hervorragende Löslichkeit in den üblichen FK-Materialien, wobei gleichzeitig das Auftreten von smektischen Phasen effektiv unterdrückt wird.

Flüssigkristalle der Formel

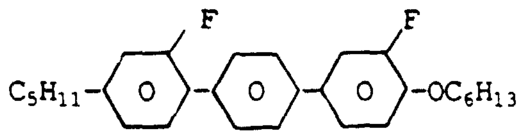

sind bereits aus der WO 8902425 bekannt. In der JP 59-155485-A2 ist eine Verbindung der Formel

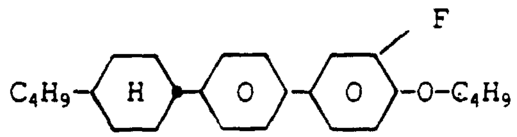

genannt. In der EP 278665 schließlich werden Verbindungen der Formel

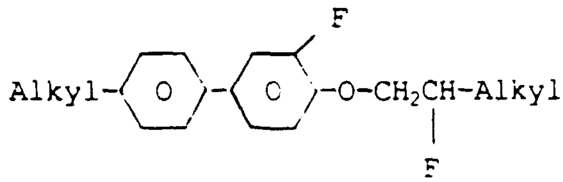

beschrieben.

Aus den DE 31 36 624, DE 32 09 178 und DE 34 10 734 sind Verbindungen der folgenden Formeln bekannt:

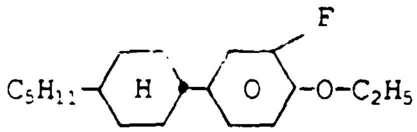

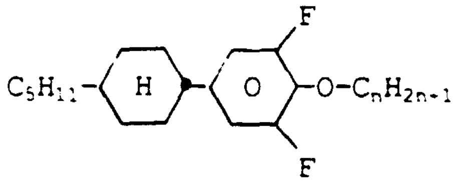

n = 1 oder 6

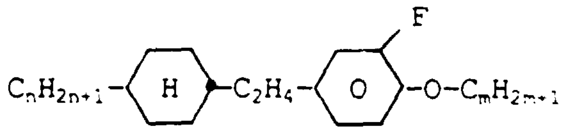

n = 5 oder 7,

m = 1,2,6,10 oder 11.

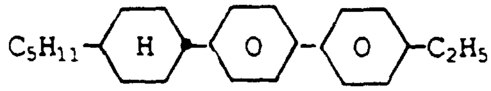

Aus der J 59 01 684 sind 3-Fluor-4-substituierte 4-Bicyclohexylbenzole der Formel

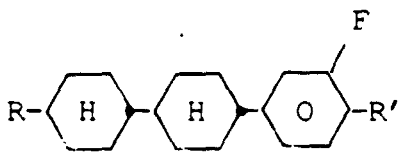

(R, R′ = $C_{1-10}$-Alkyl)

bekannt.

In der WO 88 05 803 A werden Ester der allgemeinen Formel

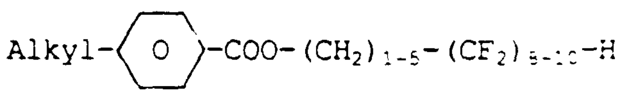

behandelt.

In der EP 0 360 521 A werden Verbindungen der allgemeinen Formel

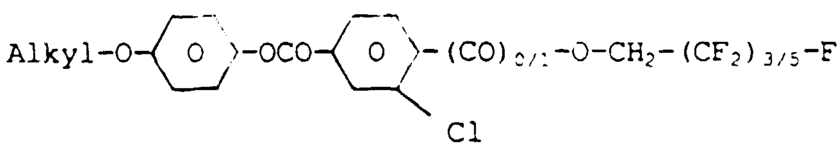

beschrieben.

Aus der WO 89 02 884 sind Trifluormethylether der Formel

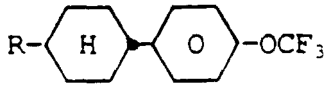

bekannt.

In der DE-OS 39 09 802 werden schließlich Verbindungen der Formel

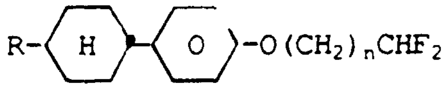

beschrieben.

Aus der EP-OS 0 168 683 sind Flüssigkristalle bekannt der folgenden Formeln:

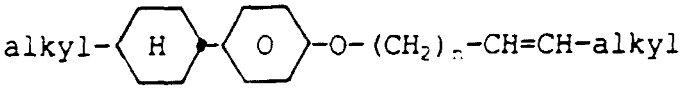

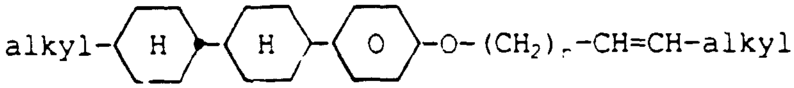

Aus der WO 90/13610 sind Verbindungen der Formel

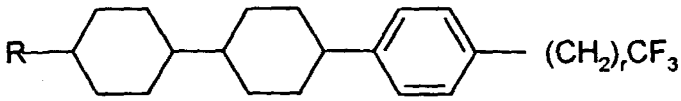

bekannt, die weder eine Ester- noch eine Etherbrücke in der Seitenkette aufweisen.

Die Verbindungen der Formel I mit Y = $CF_3$ ermöglichen sowohl STN-Anzeigen mit sehr hoher Steilheit der elektrooptischen Kennlinie als auch Anzeigen mit aktiver Matrix mit hervorragender Langzeitstabilität. Die erfindungsgemäßen Verbindungen weisen, im Vergleich zu den nicht fluorhaltigen Verbindungen, sowohl ein höheres $\Delta\varepsilon$ als auch ein höheres $\varepsilon_\perp$ auf. Aufgrund ihrer besonders günstigen elastischen Eigenschaften eignen sie sich besonders als Komponenten für TFT-Mischungen. Durch geeignete Wahl von r und s lassen sich bei beiden Anzeigetypen die Schwellenspannungen deutlich erniedrigen.

Im Hinblick auf die verschiedensten Einsatzbereiche derartiger Verbindungen war es jedoch wünschenswert, weitere Verbindungen zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen

Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Vor- und nachstehend haben R, $A^1$, A, $Z^1$, Z, W, Y, m, o, s, und r die angegebene Bedeutung, sofern nicht ausdrücklich etwas anderes vermerkt ist. Falls $A^1$ und A einen substituierten 1,4-Phenylenring bedeuten, so ist der Phenylenring vorzugsweise in 2-, 2,3- oder 2,6-Stellung durch Fluor substituiert.

$Z^1$ und Z bedeuten bevorzugt eine Einfachbindung oder $-CH_2CH_2-$.

Falls einer der Reste $Z^1$ und Z $-(CH_2)_2-$, $-(CH_2)_4-$ oder $-CH=CH-CH_2CH_2-$ bedeutet, so ist der andere Rest $Z^1$ oder Z vorzugsweise eine Einfachbindung.

Bevorzugte Verbindungen dieses Types entsprechen der Teilformel I',

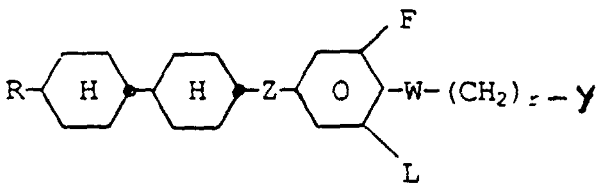

I'

worin Z $-(CH_2)_4-$ oder $-CH=CH-CH_2CH_2-$ bedeutet und R, r, L, W und Y die in Formel I angegebene Bedeutung haben.

Bevorzugt sind ebenfalls Verbindungen der Teilformel I'',

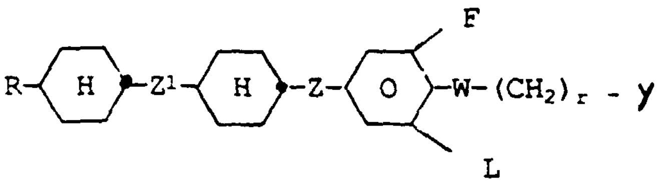

I''

worin L H oder F bedeutet und R, $Z^1$, W, Y und r die in Anspruch 1 angegebene Bedeutung besitzen.

Der Einfachheit halber bedeuten im folgenden A'' einen 1,4-Cyclohexylenrest, B einen Rest der Formel

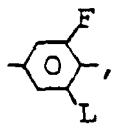

wobei L vorzugsweise F ist. Y ist vorzugsweise F, Cl oder $CF_3$ und r vorzugsweise 1, 2, 3 oder 4.

Die Verbindungen der Formel I umfassen dementsprechend Verbindungen der Teilformeln

$$R\text{-}A''\text{-}A''\text{-}B\text{-}W\text{-}(CH_2)_r\text{-}Y \qquad \text{I''a}$$

$$R\text{-}A''\text{-}Z^1\text{-}A''\text{-}B\text{-}W\text{-}(CH_2)_r\text{-}Y \qquad \text{I''c}$$

$$R\text{-}A''\text{-}A''\text{-}Z\text{ -}B\text{-}W\text{-}(CH_2)_r\text{-}Y \qquad \text{I''e}$$

$$R\text{-}A''\text{-}Z^1\text{-}A''\text{-}Z^2\text{-}B\text{-}W\text{-}(CH_2)_r\text{-}Y \qquad \text{I''g}$$

Darunter sind besonders diejenigen der Teilformeln I''a und I''e bevorzugt. Bei den Verbindungen der Teilformeln I''c bis I''h bedeuten $Z^1$ and Z $-CH_2CH_2-$, $-CH=CH-$ oder $-C{\equiv}C-$, vorzugsweise $-CH_2CH_2-$.

Besonders bevorzugt sind auch die Verbindungen der Formel IA,

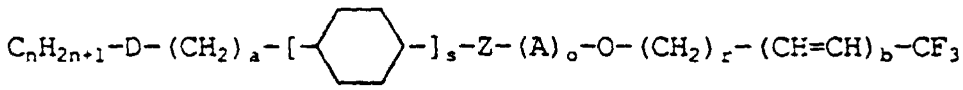 IA

worin n 0 bis 7, D -O-, -CH=CH- oder eine Einfachbindung, a 1 bis 5, b 0 bedeutet und Z, A, o, s und r die in Anspruch 1 angegebene Bedeutung besitzen.

Besonders bevorzugte Teilformeln der Verbindungen IA sind

- die Verbindungen der Formel Ia,

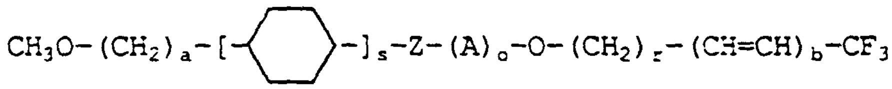 Ia

worin a 1 bis 5, b 0, s 1 oder 2 und A, Z, o und r die in Anspruch 1 angegebene Bedeutung besitzen.

- die Verbindungen der Formel Ib,

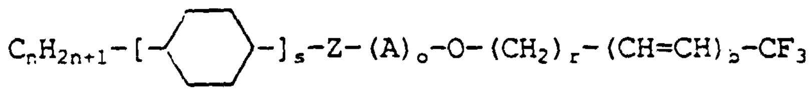 Ib

worin n 1 bis 7, s 1 oder 2, b 0 bedeuten und A, Z, o und r die in Anspruch 1 angegebene Bedeutung besitzen.

- die Verbindungen der Formel Ic,

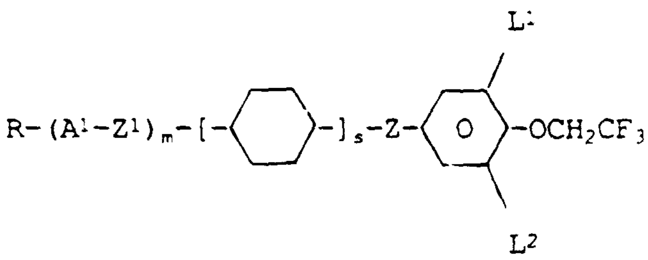 Ic

worin $L^1$ H, F oder Cl, $L^2$ F oder Cl bedeuten und R, $A^1$, $Z^1$, Z, m und s die in Anspruch 1 angegebene Bedeutung besitzen.

Falls R in Formel I einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkenylrest bedeutet, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine $CH_2$-Gruppe durch -O- und eine durch -CO- ersetzt ist, so sind

diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkenylrest bedeutet, in dem eine der Vinylgruppe benachbarte $CH_2$-Gruppe durch CO oder CO-O oder O-CO-ersetzt ist, so kann dieser geradkettig oder verzweigt sein.

Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxy- butyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R einen einfach durch CN oder $CF_3$ substituierten Alkyl- oder Alkenylrest bedeutet, so ist dieser Rest vorzugsweise geradkettig und die Substitution durch CN oder $CF_3$ in ω-Position.

Falls R einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sei, vorzugsweise jedoch in ω-Position.

Verbindungen der Formel I, die über für Polymerisationsreaktionen geeignete Flügelgruppen R verfügen, eignen sich zur Darstellung flüssigkristalliner Polymerer.

Verbindungen der Formeln I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien. Verbindungen der Formel I mit $S_A$-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr $CH_2$-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxy-carbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Unter diesen Verbindungen der Formel I sowie den Unterformeln sind diejenigen bevorzugt, in denen mindestens einer der darin enthaltenden Reste eine der angegebenen bevorzugten Bedeutungen hat.

Einige ganz besonders bevorzugte kleinere Gruppen von Verbindungen sind diejenigen der Teilformeln I1 bis I10, worin L H oder F, und r 1-7 bedeuten.

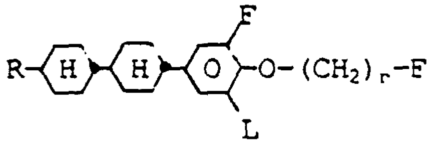

I1

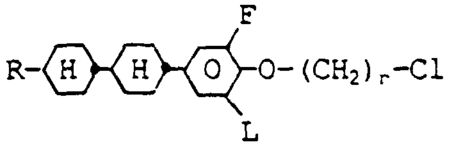

I2

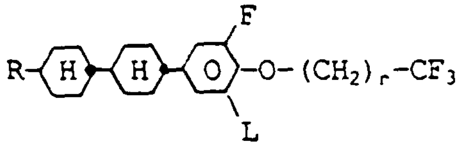

I3

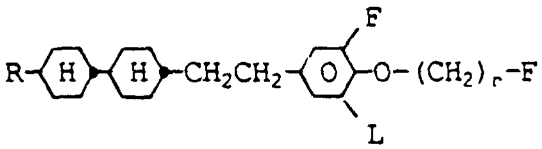

I4

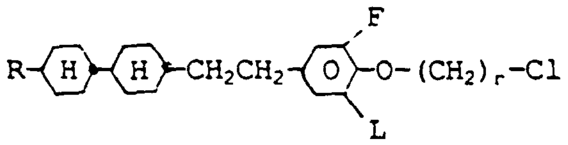

I5

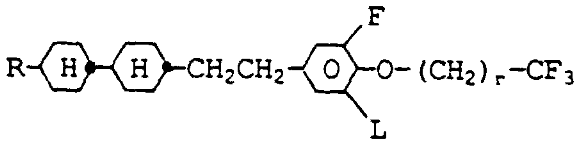

I6

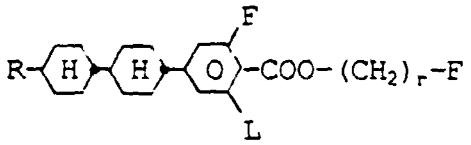

I7

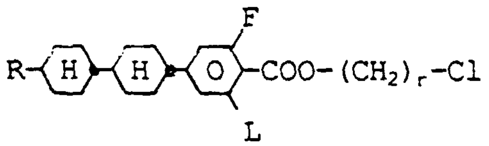

I8

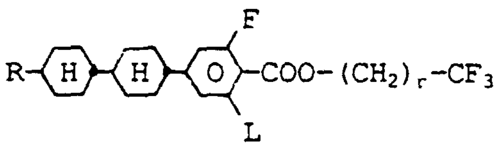

I9

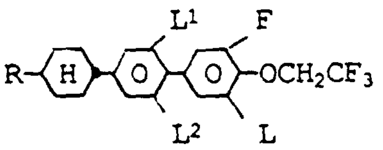

($L^1$, $L^2$ = H oder F) I 10

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd. IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die erfindungsgemäßen Verbindungen der Formel I mit W = -O- und

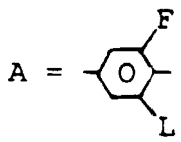

können z. B. hergestellt, indem man eine Verbindung der Formel II,

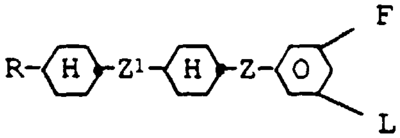

II

worin L H oder F bedeutet und R, $Z^1$ und Z die angegebene Bedeutung haben, gemäß folgendem Reaktionsschema metalliert und anschließend mit einem geeigneten Elektrophil umsetzt:

<u>**Schema 1**</u>

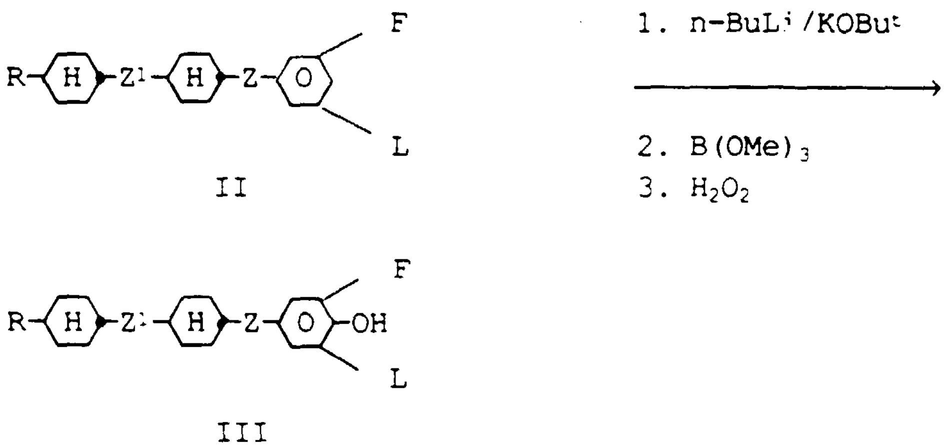


Die Verbindungen der Formel II mit L = F sind neu und somit auch Gegenstand der Erfindung. Weiterhin sind Verbindungen der Formel III mit L = F Gegenstand der Erfindung.

Gegenstand der Erfindung sind ferner flüssigkristalline Medien mit einem Gehalt an mindestens einer Verbindung der Formel II.

Aus dem erhaltenen Phenol der Formel III sind die Verbindungen der Formel I (W = -O-) (W = -O- und

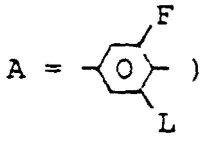

nach bekannten Veretherungs-Methoden, z.B. durch Umsetzung mit Hal-$(CH_2)_r$-Q-$(CH_2)_t$-Y (Hal = I, Br oder Cl) in Aceton/$K_2CO_3$, ggf. in Gegenwart von katalytischen Mengen KI, erhältlich.

Weitere Synthesemethoden für die erfindungsgemäßen Phenylether sind für den Fachmann augenscheinlich. Beispielsweise können in 5-Position entsprechend substituierte 1,3-Difluorbenzol-Verbindungen oder monofluorierte Analoga (L = H) gemäß obigem Schema in die 2-O-$(CH_2)_r$-Y-1,3 - difluor - Verbindungen oder monofluorierte Analoga (L = H) überführt werden und der Rest

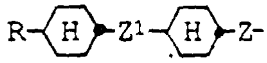

anschließend durch in der Flüssigkristallchemie gebräuchliche Reaktionen (z.B. Veretherung oder Kopplungen z.B. gemäß der Artikel E. Poetsch, Kontakte (Darmstadt) 1988 (2), S. 15) eingeführt werden.

Die Verbindungen der Formel II können beispielsweise nach folgendem Syntheseschema hergestellt werden:

<u>**Schema 2**</u>

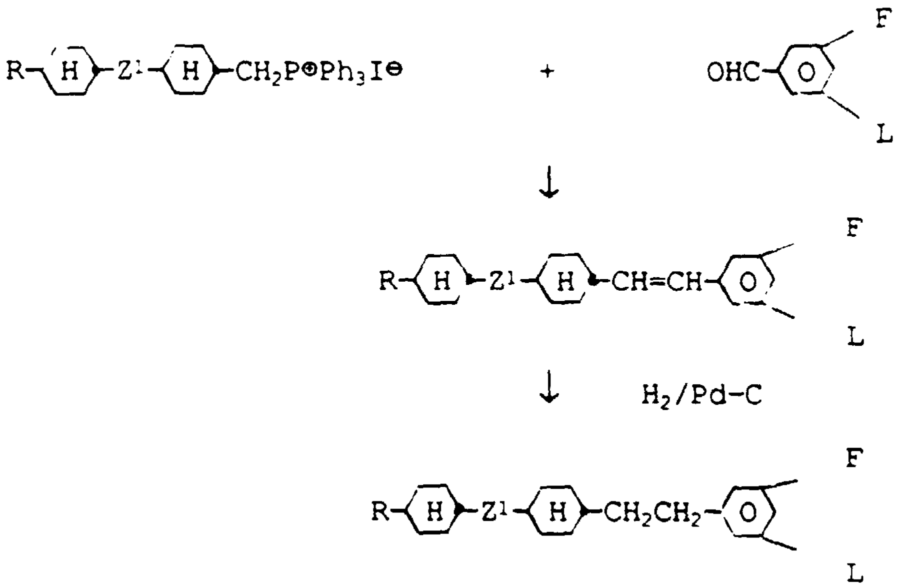


Die Synthese einiger besonders bevorzugter Verbindungen ist im folgenden näher angegeben:

**Schema 3** (L = H oder F)

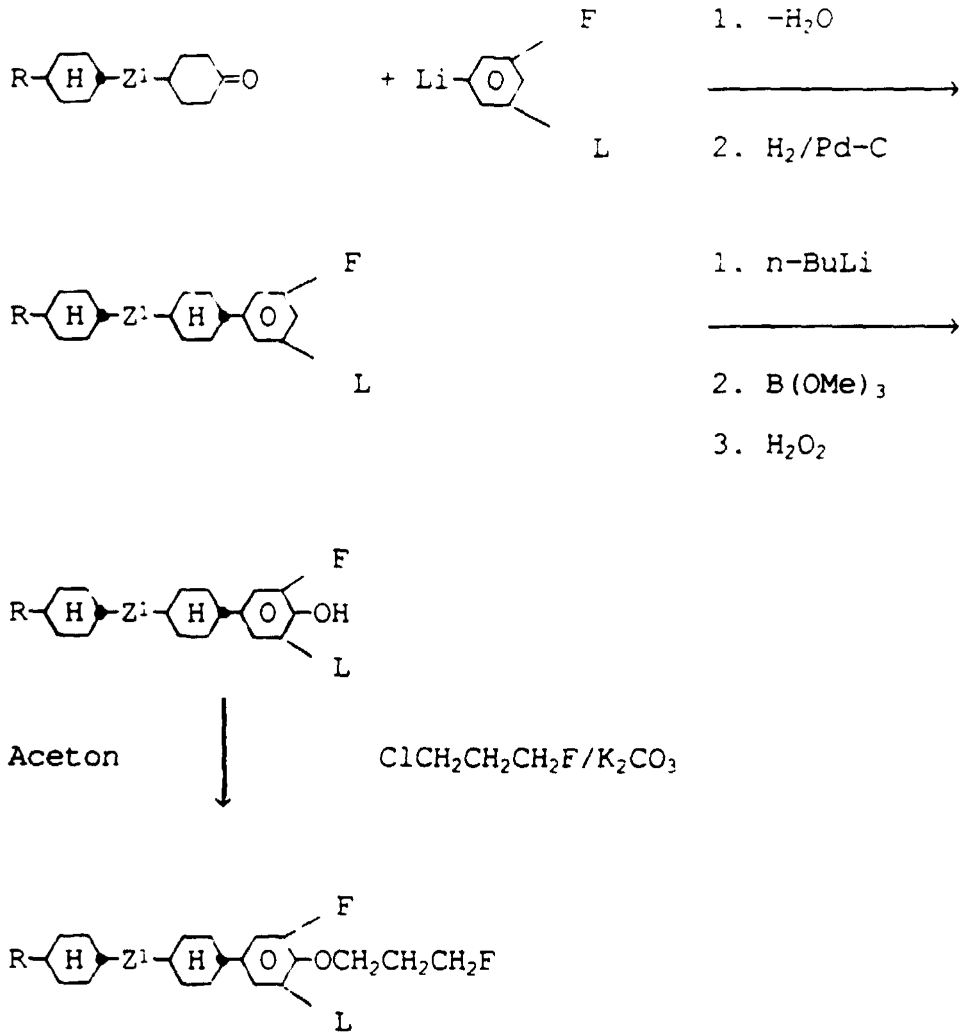

Schema 4

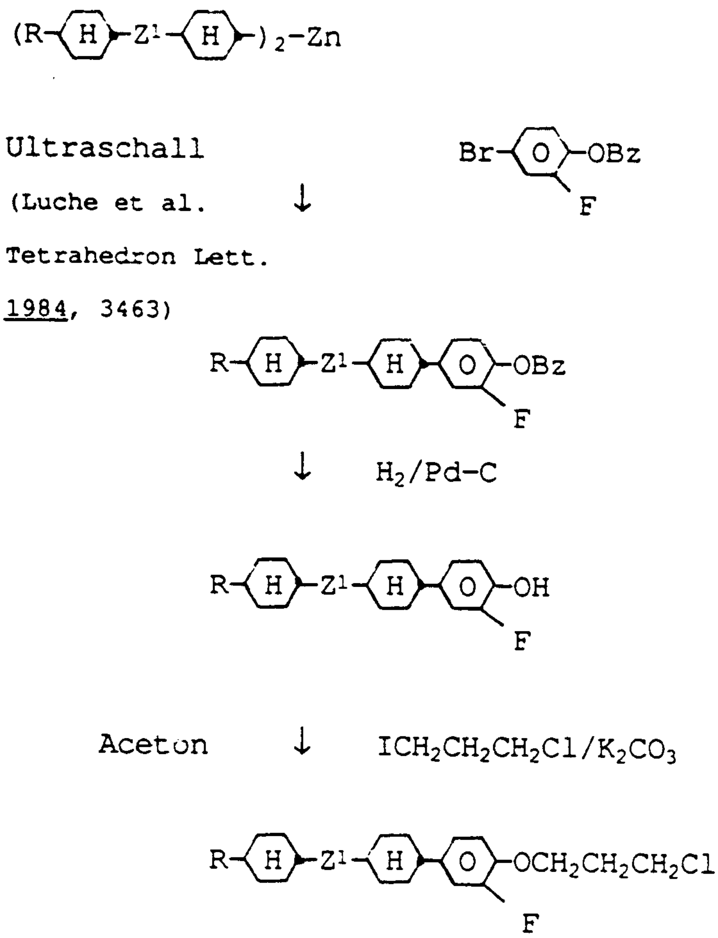


Phenylether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z. B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20 und 100 °C.

Weiterhin können die Alkylether auch nach Mitsunobu aus den entsprechenden Phenolen und einer Hydroxyverbindung in Gegenwart von Triphenylphosphin und Diethylazodicarboxylat hergestellt werden (S. Bittner, Y. Assaf, Chemistry and Industry, 281 (1975); M.S. Manhas, W.H. Hoffman, B. Lal, A.K. Bose, J. Chem. Soc. Perkin I, 461 (1975)):

**Schema 5**

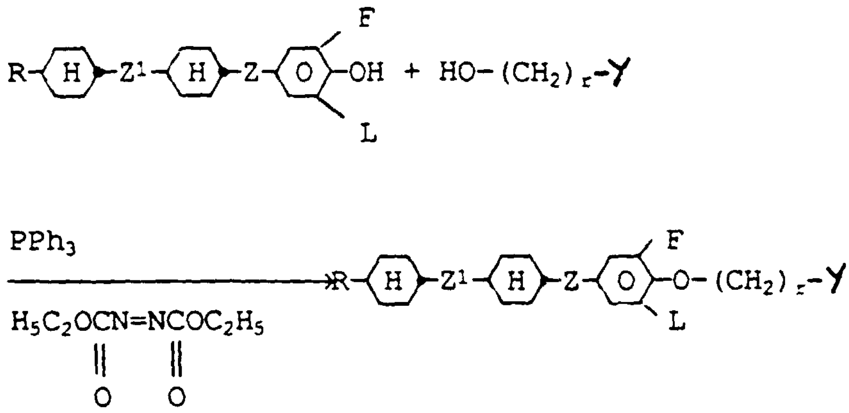


Die Ausgangsmaterialien für die Herstellungsverfahren sind entweder bekannt oder können in Analogie zu bekannten Verbindungen hergestellt werden.

Die Verbindungen der Formel I''' mit Z = $(CH_2)_4$- können nach folgendem Schema hergestellt werden (r = 1-7):

**Schema 6** (L = H oder F)

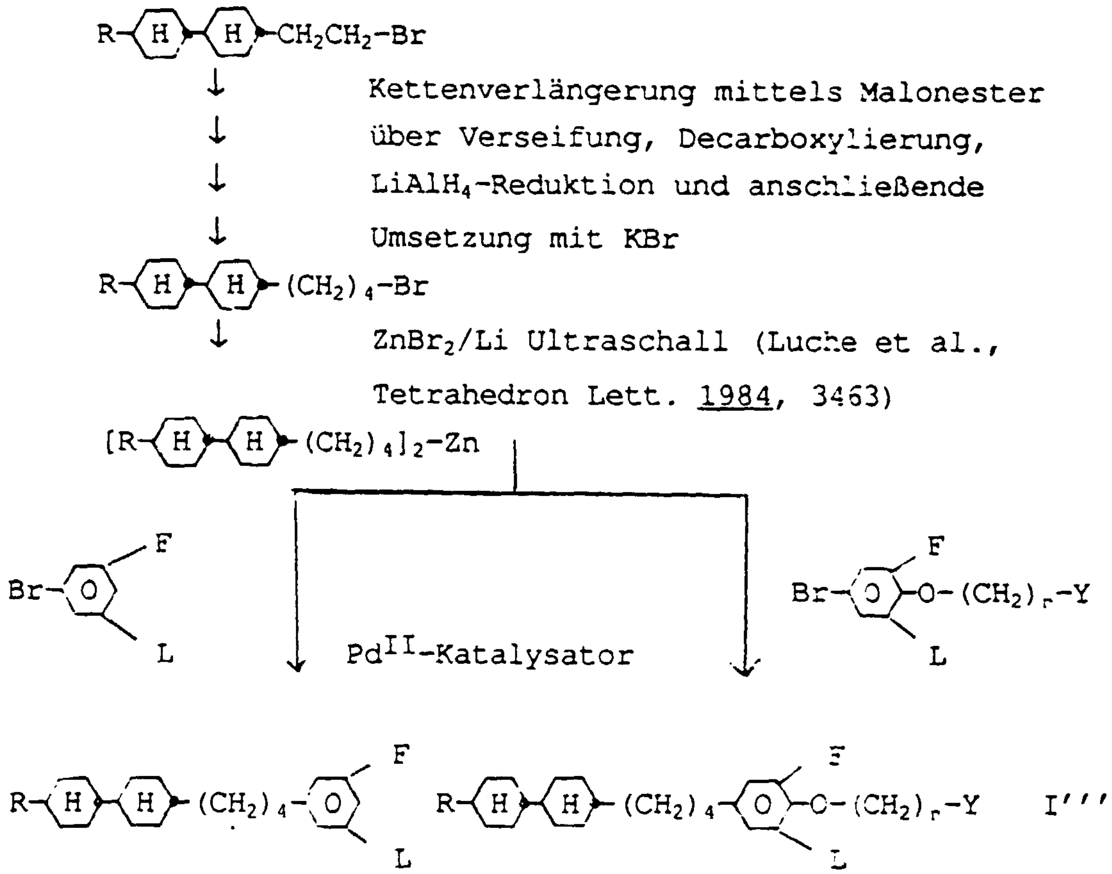


I'''

Bei der Pd(II)-katalysierten Kopplungsreaktion wird entweder direkt das Zielprodukt I''' gebildet oder ein Vorprodukt, in das völlig analog zu den vorstehenden Methoden für Verbindungen der Formel I der Rest $-O-(CH_2)_r-Y$ eingeführt wird.

Die erfindungsgemäßen Phenylester der Formel I (Q = -COO-) erhält man aus den Verbindungen der Formel II gemäß folgendem Reaktionsschema:

**Schema 7** (L = H oder F)

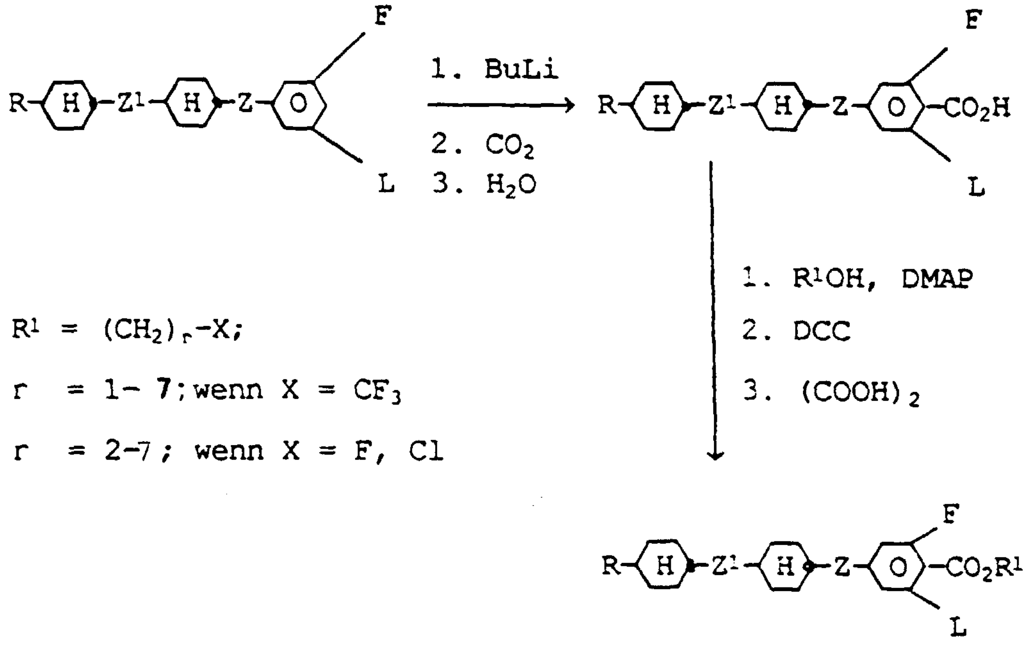


Die Trifluoralkylether der Formel

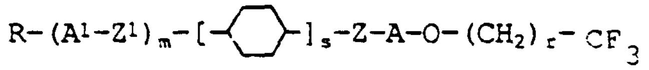

sind nach bekannten Veretherungsmethoden, z.B. durch Umsetzung von 4-substituierten Phenolen mit Hal-$(CH_2)_r$-$CF_3$, wobei Hal I, Br oder Cl bedeutet, in Aceton und Kaliumcarbonat gegebenenfalls in Gegenwart von katalytischen Mengen Kaliumiodid erhältlich.

Die Veretherung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Kohlenwasserstoffe wie Benzol, Toluol oder Xylol, Halogenkohlenwasserstoffe wie Tetrachlorkohlenstoff, Dichlormethan oder Tetrachlorethylen.

Die Synthese der erfindungsgemäßen Trifluoralkyl-Verbindungen ist den folgenden Schemata zu entnehmen:

Schema 8

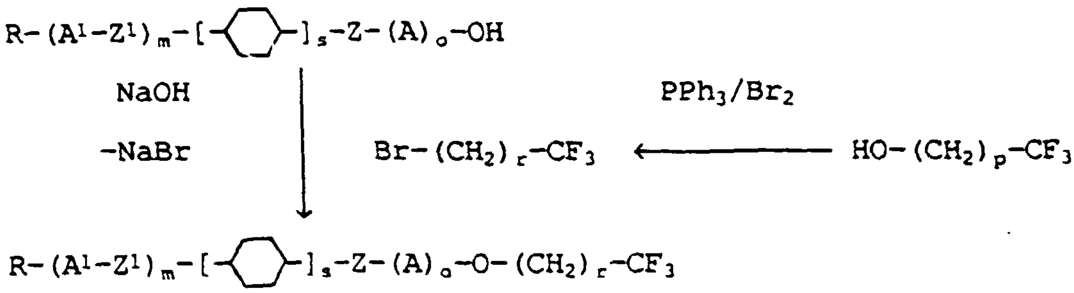


Schema 9

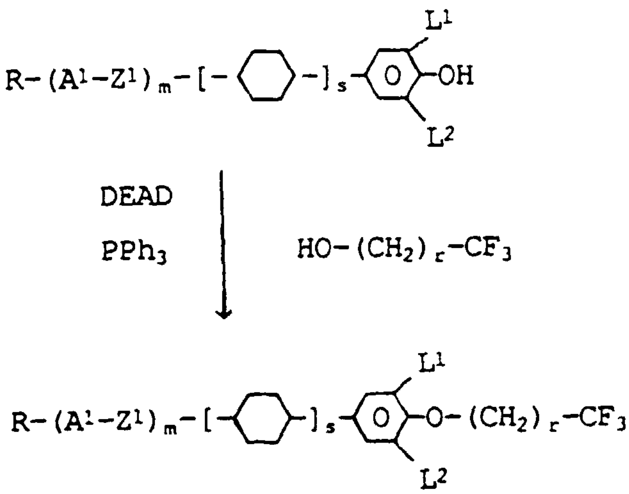


($L^1$, $L^2$: H oder F)

Die Verbindungen der Formel

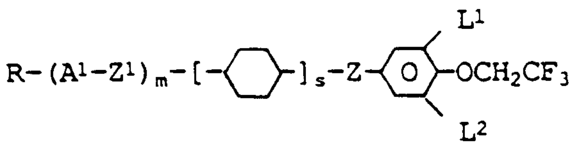

lassen sind wie folgt herstellen:

**Schema 10** (L = H oder F)

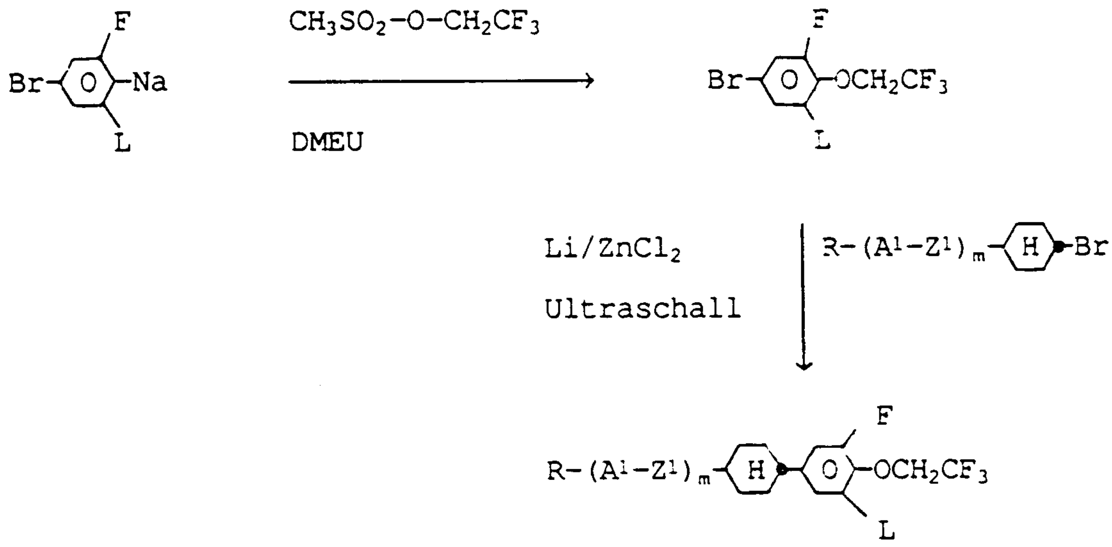


**Schema 11**

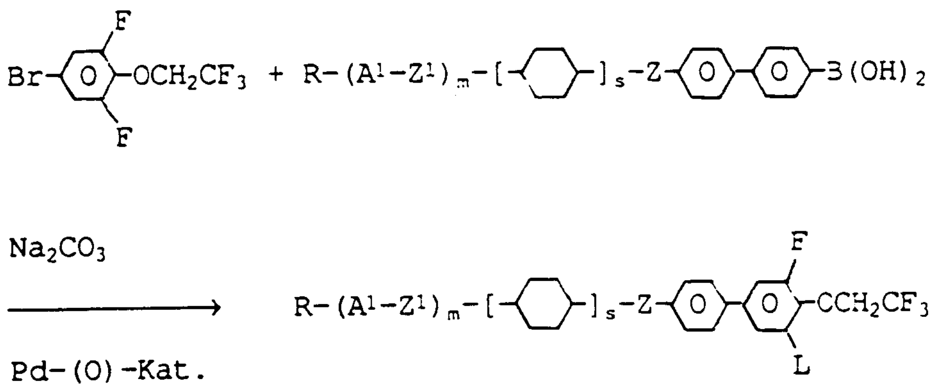


Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäure-phenyl- oder cyclohexylester, Phenyl- oder Cyclohexyl-ester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-$CH_2CH_2$-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2-5-diyl oder Pyridin-2,5-diyl, Bio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder $-(O)_i\, CH_{3-(k+1)}\, F_kCl_1$, wobei i 0 oder 1 und k+1 1, 2 oder 3 sind; die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R" die Bedeutung -F, -Cl, -NCS, $-CF_3$, $-OCHF_2$ oder $-OCF_3$ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1c, 2c, 3c, 4c und 5 c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise

Gruppe A: 0 bis 90 %, vorzugsweise 20 bis 90 %, insbesondere 30 bis 90 %

Gruppe B: 0 bis 80 %, vorzugsweise 10 bis 80 %, insbesondere 10 bis 65 %

Gruppe C: 0 bis 80 %, vorzugsweise 5 bis 80 %, insbesondere 5 bis 50 %
wobei die Summe der Massenanteile der in den jeweiligen erfindungsgemäßen Medien enthaltenen Verbindungen aus den Gruppen A und/oder B und/oder C vorzugsweise 5 bis 90 % und insbesondere 10 bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie. Vor- und nachstehend bedeuten Prozentgaben Gewichtsprozent. Alle Temperaturen sind in Grad Celsius angegeben. Fp. bedeutet Schmelzpunkt und Kp. = Klärpunkt. Ferner bedeuten K = kristalliner Zustand, N = nematische Phase S = smektische Phase und I = isotrope Phase. Die Angaben zwischen diesen Symbolen stellen die Übergangstemperaturen dar. $\Delta n$ bedeutet optische Anisotropie (589 nm, 20 °C). Die Viskosität ($mm^2$/sec) wurde bei 20 °C bestimmt.

Folgende Abkürzungen werden verwendet:

| | |
|---|---|
| BuLi | Butyllithium |
| DAST | Diethylaminoschwefeltrifluorid |
| DCC | Dicyclohexylcarbodiimid |
| DIBALH | Diisobutylaluminiumhydrid |
| DMAP | 2-Dimethylaminopyridin |
| DDQ | Dichloriddicyanobenzochinon |
| KOT | Kalium-tertiär-butanolat |
| $NH_4Cl$ | Ammoniumchlorid |
| THF | Tetrahydrofuran |
| TMEDA | Tetramethylethylendiamin |
| pTSOH | p-Toluolsulfonsäure |

<u>Vergleichsbeispiel</u>

a) 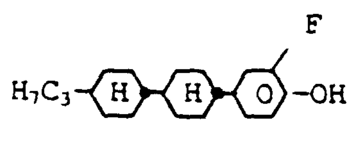

Zu einem Gemisch bestehend aus 26 mmol 1-trans-4-(trans-4-n-Propylcyclohexyl)-cyclohexyl-3-fluorbenzol (hergestellt nach Schema 1), 4,1 g Kalium-tert.-butylat und 60 ml THF werden bei -100 °C 26 mmol n-BuLi getropft. Nach einer Stunde Rühren bei -100 °C werden bei -85 bis -90 °C 36 mmol Borsäuretrimethylester zugetropft. Es wird noch 0,5 h nachgerührt, und dann werden bei -20 °C 42 mmol Essigsäure zugetropft. Anschließend erwärmt man auf 30 °C und tropft bei dieser Temperatur 4,2 ml $H_2O_2$ zu und läßt zwei Stunden bei 50 bis 60 °C rühren. Man läßt auf Raumtemperatur abkühlen und versetzt das Gemisch mit einer 5%igen Natriumdithionlösung. Nach Phasentrennung und üblicher Aufarbeitung erhält man das Phenol.

b) 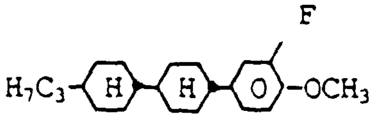

Das erhaltene Phenol wird mit Methyliodid in Aceton unter Rückfluß in Gegenwart von Kaliumcarbonat zum Methylether umgesetzt. Nach üblicher Aufarbeitung und Chromatographie an Kieselgel mit Hexan erhält man den Ether in reiner Form.

Analog werden die folgenden Ether der Formel

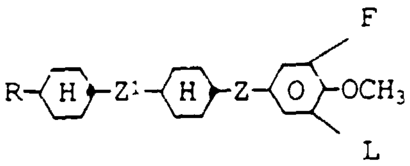

hergestellt.

| R | 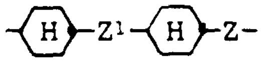 | L |
|---|---|---|
| $CH_3$ | 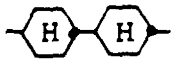 | H |
| $C_2H_5$ | 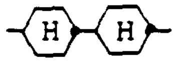 | H |
| n-$C_5H_{11}$ | 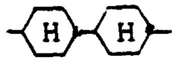 | H |
| $CH_3OCH_2$ | 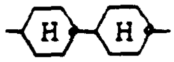 | H |
| $CH_2$=$CHCH_2CH_2$ | 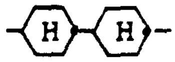 | H |
| $CH_3$ | 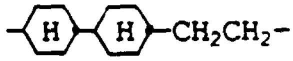 | H |
| $C_2H_5$ | 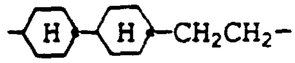 | H |
| n-$C_3H_7$ | 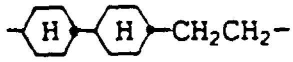 | H |
| n-$C_5H_{11}$ | 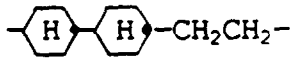 | H |
| $CH_3OCH_2$ | 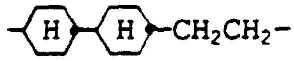 | H |
| $CH_2$=$CHCH_2CH_2$ | 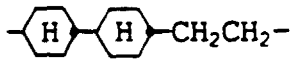 | H |
| $CH_3$ | 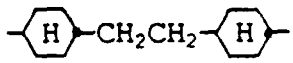 | H |
| $C_2H_5$ | 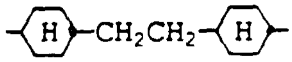 | H |
| n-$C_3H_7$ | 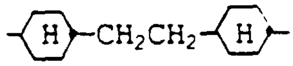 | H |
| n-$C_5H_{11}$ | 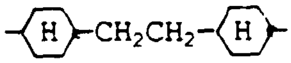 | H |
| $CH_3OCH_2$ | 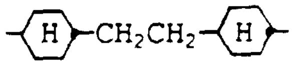 | H |
| $CH_2$=$CHCH_2CH_2$ | 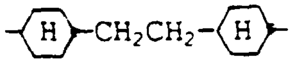 | H |
| n-$C_5H_{11}$ | 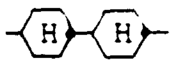 | F K 43 N 154,3 I |

| R | 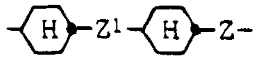 |
|---|---|
| $CH_3$ | 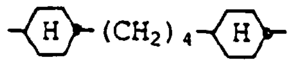 |
| $C_2H_5$ | 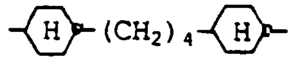 |
| $n-C_3H_7$ | 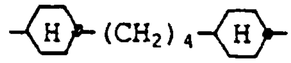 |
| $n-C_5H_{11}$ | 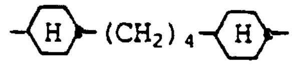 |
| $CH_3OCH_2$ | 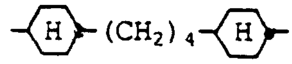 |
| $CH_2=CHCH_2CH_2$ | 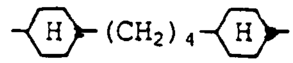 |

**Beispiel 1**

a) 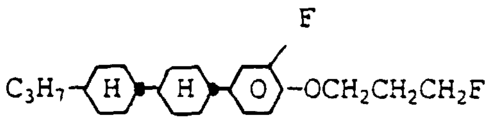

Das in Beispiel 1 a) hergestellte Fluorphenol wird mit 1-Chlor-3-fluor-propan in siedendem Aceton in Gegenwart von Kaliumcarbonat und einer katalytischen Menge Kaliumiodid zum Phenolether umgesetzt. Nach üblicher Aufarbeitung und Chromatographie an Kieselgel mit Hexan erhält man des Ether in reiner Form.

b) 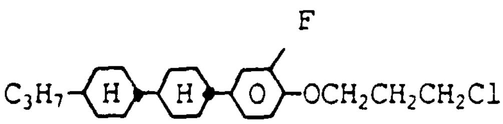

Das in Beispiel 1 a) hergestellte Fluorphenol wird mit 1- Chlor-3-Iodpropan analog Beispiel 2 a) in den Phenolether überführt.

Analog erhält man aus den entsprechenden Vorstufen die folgenden erfindungsgemäßen Verbindungen der Formel

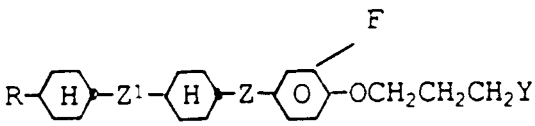

| R | 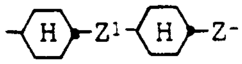 | Y |
|---|---|---|
| $CH_3$ | 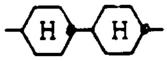 | F |
| $CH_3$ | 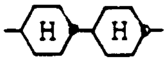 | Cl |
| $C_2H_5$ | 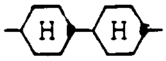 | F |
| $C_2H_5$ | 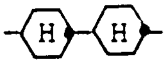 | Cl |
| $n-C_4H_9$ | 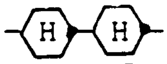 | F |
| $n-C_4H_9$ | 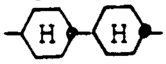 | Cl |
| $n-C_5H_{11}$ | 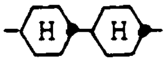 | F |
| $n-C_5H_{11}$ | 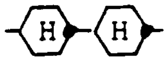 | Cl |
| $CH_3OCH_2$ | 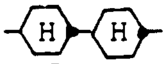 | F |
| $CH_3OCH_2$ | 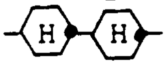 | Cl |
| $CH_2=CHCH_2CH_2$ | 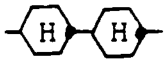 | F |
| $CH_2=CHCH_2CH_2$ | 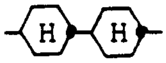 | Cl |
| $CH_3$ | 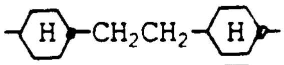 | F |
| $CH_3$ | 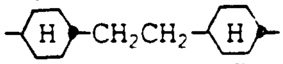 | Cl |
| $C_2H_5$ | 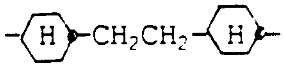 | F |
| $C_2H_5$ | 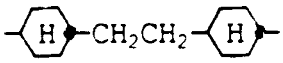 | Cl |
| $n-C_3H_7$ | 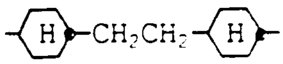 | F |
| $n-C_3H_7$ | 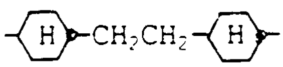 | Cl |

| R | 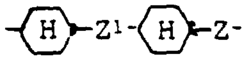 | Y |
|---|---|---|
| n-$C_4H_9$ | 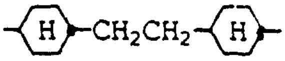 | F |
| n-$C_4H_9$ | 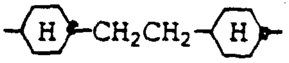 | Cl |
| n-$C_5H_{11}$ | 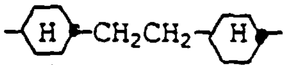 | F |
| n-$C_5H_{11}$ | 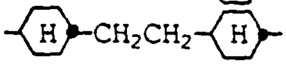 | Cl |
| $CH_3OCH_2$ | 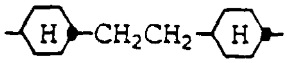 | F |
| $CH_3OCH_2$ | 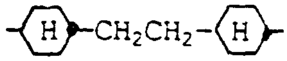 | Cl |
| $CH_2$=$CHCH_2CH_2$ | 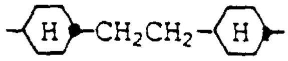 | F |
| $CH_2$=$CHCH_2CH_2$ | 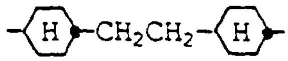 | Cl |

**Beispiel 2**

a) 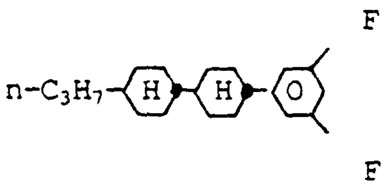

Zu einer Lösung von 6,0 g Magnesiumspänen in 60 ml Ether gibt man zwei Tropfen Brom. Anschließend wird die Lösung von 48,2 g 3,5-Difluorbrombenzol in 60 ml Ether zugetropft. Man rührt 0,5 h nach und tropft dann bei 20-25 °C eine Lösung von 44,5 g 4-trans-(4-Propylcyclohexyl)-cyclohexanon in 50 ml Ether zum Grignardreagenz. Es wird zwei Stunden nachgerührt, auf 500 ml Wasser gegossen, mit 30 ml konz. Salzsäure angesäuert und mit Ether ausgeschüttelt. Die organische Phase wird zum Rückstand eingedampft und anschließend mit 1000 ml Toluol und 120 ml 20%iger Schwefelsäure 1 h am Rückfluß gekocht.

Nach Phasentrennung und Neutralisation mit gesättigter Natriumhydrogencarbonat-Lösung wird mit 10 g Pd/C (5 %) bei 1 bar und 60 °C hydriert. Anschließend wird filtriert und eingeengt. Nach flash Chromatographie erhält man das reine Produkt. K 60° N 87,6° I, $\Delta\varepsilon$ = 3,3

b) 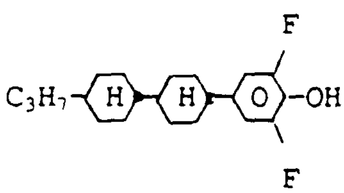

Zu einem Gemisch aus 47 mmol 4-trans-(4-n-Propylcyclohexyl)-cyclohexyl-1-trans-(3,5-difluorbenzol), 50 mmol TMEDA und 150 ml THF werden bei -65 °C bis -70 °C 31 ml n-BuLi (15 % in Hexan) zugetropft und es wird eine

Stunde bei -70 °C nachgerührt. Dann werden bei -85 bis -90 °C zunächst 57 mmol Borsäuretrimethylester, dann bei -20 °C 65 mmol Essigsäure zugetropft.

c) 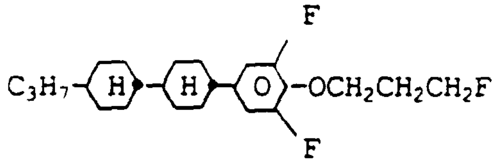

Analog Beispiel 2 a) wird das Difluorphenol mit 1-Chlor-3-fluorpropan zum Phenolether umgesetzt.

d) 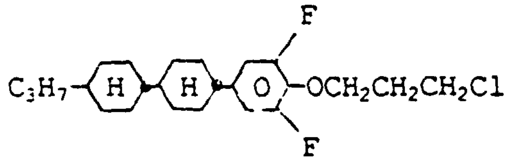

Analog Beispiel 2 b) wird das in Beispiel 4 b) hergestellte Difluorphenol mit 1-Chlor-3-Iod-propan umgesetzt. K 50 N 157,2 I, Δε = 5,7

**Beispiel 3**

a) 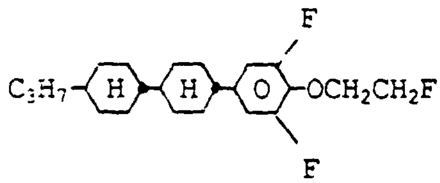

Zu einer Lösung bestehend aus 1 mmol 4-trans-(4-n-Propylcyclohexyl)-cyclohexyl-1-trans-(3,5-difluorphenol), 1 mmol Triphenylphosphin, 1 mmol 2-Fluorethanol und 25 ml THF wird 1 mmol Diethylazodicarboxylat bei 0-10 °C zugetropft. Anschließend wird noch eine Stunde bei Raumtemperatur nachgerührt und dann wie üblich aufgearbeitet. K 82 N 165,5 I, Δε = 2,07

Analog erhält man aus den entsprechenden Vorstufen die folgenden erfindungsgemäßen Verbindungen der Formel

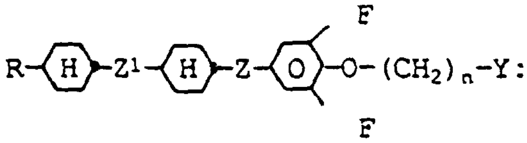

| R | 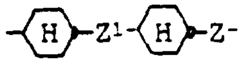 | n | Y |
|---|---|---|---|
| $CH_3$ | 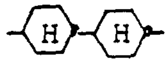 | 1 | F |
| $CH_3$ | 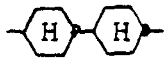 | 1 | Cl |
| $CH_3$ | 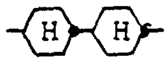 | 2 | F |
| $CH_3$ | 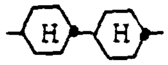 | 2 | Cl |
| $CH_3$ | 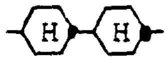 | 3 | F |
| $CH_3$ | 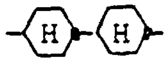 | 3 | Cl |
| $CH_3$ | 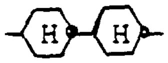 | 4 | F |
| $CH_3$ | 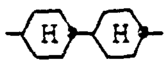 | 4 | Cl |
| $CH_3$ | 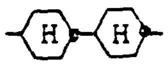 | 5 | F |
| $CH_3$ | 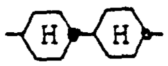 | 5 | Cl |
| $C_2H_5$ | 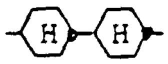 | 1 | F |
| $C_2H_5$ | 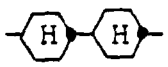 | 1 | Cl |
| $C_2H_5$ | 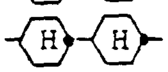 | 2 | F |
| $C_2H_5$ | 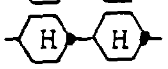 | 2 | Cl |
| $C_2H_5$ | 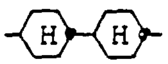 | 3 | F |
| $C_2H_5$ | 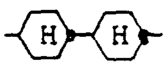 | 3 | Cl |
| $C_2H_5$ | 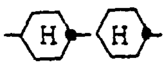 | 4 | F |
| $C_2H_5$ | 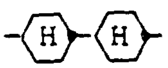 | 4 | Cl |
| $C_2H_5$ | 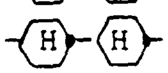 | 5 | F |
| $C_2H_5$ | 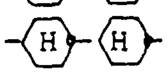 | 5 | Cl |

| R | 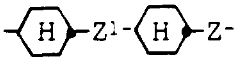 | n | Y | |
|---|---|---|---|---|
| n-$C_3H_7$ | 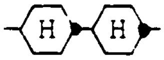 | 1 | F | |
| n-$C_3H_7$ | 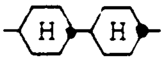 | 1 | Cl | |
| n-$C_3H_7$ | 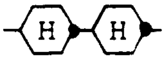 | 2 | Cl | |
| n-$C_3H_7$ | 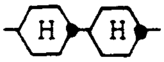 | 2 | F | K 82 N 165,5 I |
| n-$C_3H_7$ | 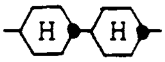 | 3 | Cl | |
| n-$C_3H_7$ | 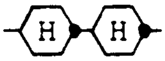 | 4 | F | |
| n-$C_3H_7$ | 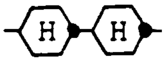 | 4 | Cl | |
| n-$C_3H_7$ | 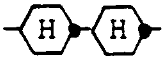 | 5 | F | |
| n-$C_3H_7$ | 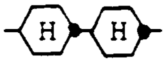 | 5 | Cl | |
| n-$C_4H_9$ | 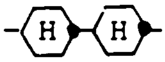 | 2 | F | |
| n-$C_4H_9$ | 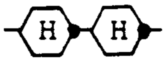 | 2 | Cl | |
| n-$C_4H_9$ | 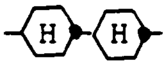 | 3 | F | |
| n-$C_4H_9$ | 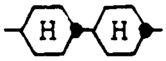 | 3 | Cl | |
| n-$C_5H_{11}$ | 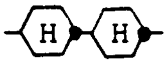 | 1 | F | |
| n-$C_5H_{11}$ | 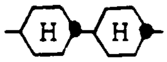 | 1 | Cl | |
| n-$C_5H_{11}$ | 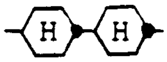 | 2 | F | |
| n-$C_5H_{11}$ | 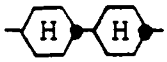 | 2 | Cl | |
| n-$C_5H_{11}$ | 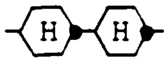 | 3 | F | |
| n-$C_5H_{11}$ | 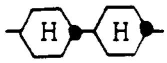 | 3 | Cl | |
| n-$C_5H_{11}$ | 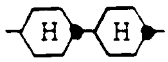 | 4 | F | |
| n-$C_5H_{11}$ | 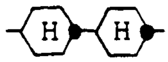 | 4 | Cl | |
| n-$C_5H_{11}$ | 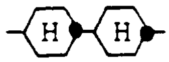 | 5 | F | |
| n-$C_5H_{11}$ | 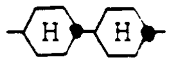 | 5 | Cl | |

| R | 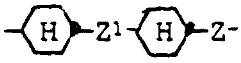 | n | Y |
|---|---|---|---|
| $CH_3OCH_2$ | 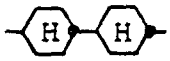 | 2 | F |
| $CH_3OCH_2$ | 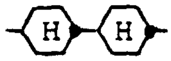 | 2 | Cl |
| $CH_3OCH_2$ | 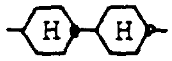 | 3 | F |
| $CH_3OCH_2$ | 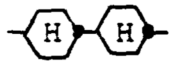 | 3 | Cl |
| $CH_2{=}CHCH_2CH_2$ | 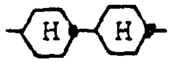 | 2 | F |
| $CH_2{=}CHCH_2CH_2$ | 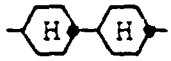 | 2 | Cl |
| $CH_2{=}CHCH_2CH_2$ | 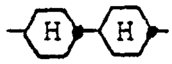 | 3 | F |
| $CH_2{=}CHCH_2CH_2$ | 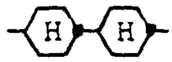 | 3 | Cl |

**Beispiel 4**

a) 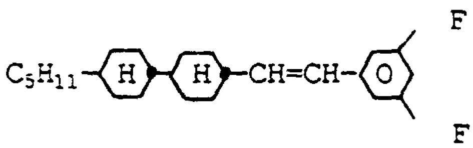

Zu 78 mmol 1-(4-(4-n-Pentylcyclohexyl)-cyclohexyl)methylentriphenylphosphoniumiodid und 78 mmol 3,5-Difluorbenzaldehyd gelöst in 50 ml THF werden bei -5 °C 78 mmol Kalium-tert.-butylat gelöst in 80 ml THF zugetropft. Anschließend wird bei gleicher Temperatur 15 Minuten nachgerührt und weitere 1,5 h bei Raumtemperatur. Das Reaktionsgemisch wird hydrolysiert und mit Salzsäure neutralisiert. Anschließend wird wie üblich aufgearbeitet.

b) 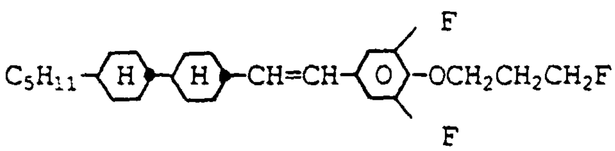

Analog Beispiel 3 c) wird das in Beispiel 5 a) hergestellte Produkt mit 1-Chlor-3-fluor-propan zum Phenolether umgesetzt.

c) 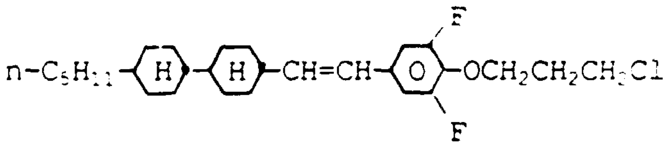

Analog Beispiel 3 d) wird das in Beispiel 5 a) hergestellte Produkt mit 1-Chlor-3-iod-propan zum Phenolether umgesetzt.

Analog erhält man aus den entsprechenden Vorstufen der Formel II (L = F) die folgenden erfindungsgemäßen Verbindungen der Formel

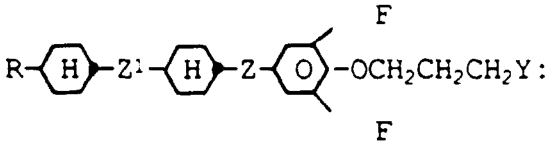

| R | 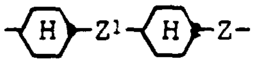 | Y |
|---|---|---|
| $CH_3$ | 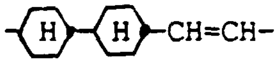 | F |
| $CH_3$ | 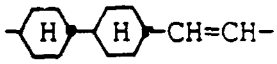 | Cl |
| $C_2H_5$ | 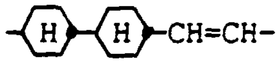 | F |
| $C_2H_5$ | 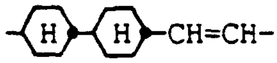 | Cl |
| $n-C_3H_7$ | 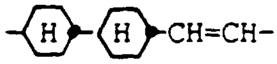 | F |
| $n-C_3H_7$ | 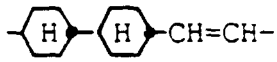 | Cl |
| $n-C_4H_9$ | 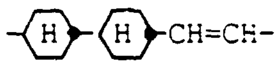 | F |
| $n-C_4H_9$ | 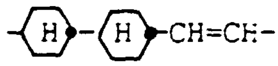 | Cl |
| $CH_3OCH_2$ | 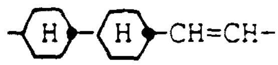 | F |
| $CH_3OCH_2$ | 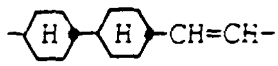 | Cl |
| $CH_2=CHCH_2CH_2$ | 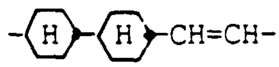 | F |
| $CH_2=CHCH_2CH_2$ | 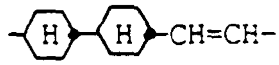 | Cl |

**Beispiel 5**

a) 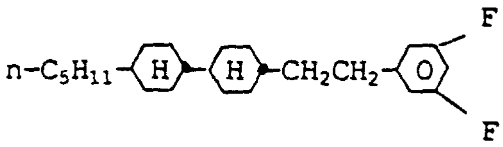

9 mmol Produkt aus Beispiel 5 a) werden in 30 ml THF gelöst, mit 0,3 g 4 % Pd-C versetzt und hydriert. Anschließend wird vom Katalysator abfiltriert und das Filtrat im Vakuum zum Rückstand eingeengt. Dieser wird über eine Kieselgelsäule mit Pentan chromatographiert.

Analog erhält man

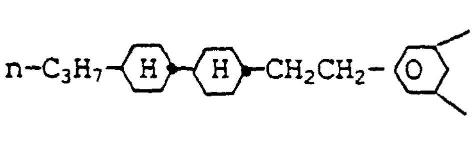 , K 52 N 95,1 I, $\Delta\varepsilon$ = 4,7.

b) 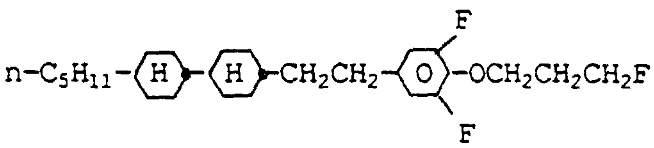

Analog Beispiel 3 c) wird das in Beispiel 6 a) gewonnene Produkt mit 1-Chlor-3-fluor-propan umgesetzt.

c) 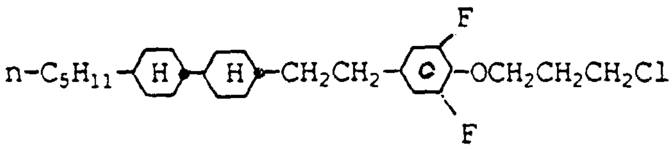

Analog Beispiel 3 d) wird das in Beispiel 6 a) hergestellte Produkt mit 1-Chlor-3-iod-propan zum Phenolether umgesetzt.

Analog erhält man aus den entsprechenden Vorstufen der Formel II (L = F) die folgenden Verbindungen der Formel

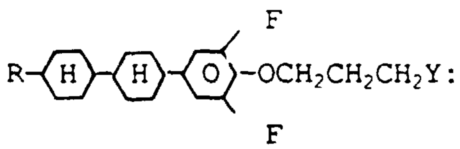

| R | 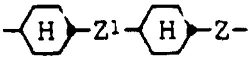 | Y |
|---|---|---|
| $CH_3$ | 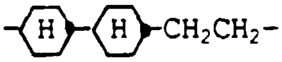 | F |
| $CH_3$ | 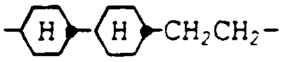 | Cl |
| $C_2H_5$ | 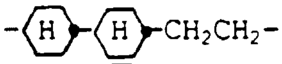 | F |
| $C_2H_5$ | 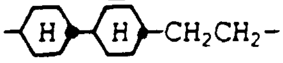 | Cl |
| $n-C_3H_7$ | 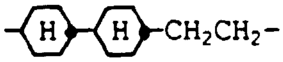 | F |
| $n-C_3H_7$ | 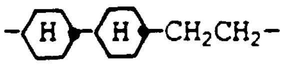 | Cl |
| $n-C_4H_9$ | 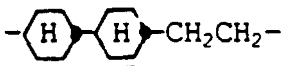 | F |
| $n-C_4H_9$ | 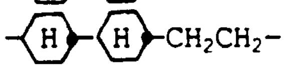 | Cl |
| $CH_3OCH_2$ | 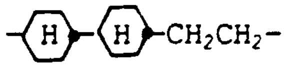 | F |
| $CH_3OCH_2$ | 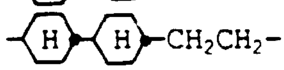 | Cl |
| $CH_2=CHCH_2CH_2$ | 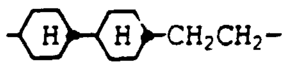 | F |
| $CH_2=CHCH_2CH_2$ | 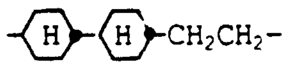 | Cl |

**Beispiel 6**

a) 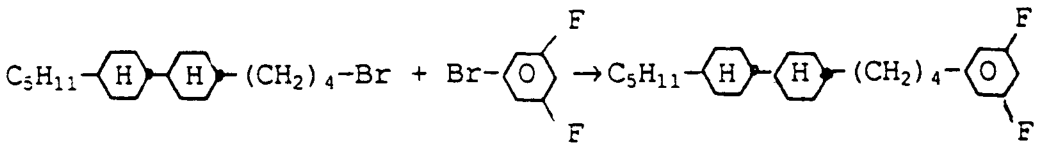

0,1 mol 4-(4-n-Pentylcyclohexyl)-(cyclohexylbutylbromid) werden in 150 ml eines Lösungsmittelgemisches von THF/Toluol (1:4) vorgelegt, dann 11,5 g Zinkbromid wasserfrei und darauf 1,4 g Lithiumgranulat hinzugefügt. Das Gemisch wird unter Argon und Rühren 4 Stunden zwischen 0 °C und 10 °C mit Ultraschall behandelt. Die entstehende zinkorganische Verbindung wird mit 0,1 mol 3,5-Difluor-1-brom-benzol und 2 mol% 1,1'-Bis(diphenylphosphino)-ferrocen-palladium(II)dichlorid versetzt und nach Entfernung des Ultraschallbades und der Kühlung 24 h bei Zimmertemperatur gerührt. Es wird mit 100 ml gesättigter $NH_4Cl$-Lösung unter Rühren zersetzt, die organische Phase abgetrennt, die wäßrige Phase mit Toluol extrahiert. Die vereinigten organischen Extrakte werden getrocknet, eingeengt und über Kieselgel chromatographiert.

b) 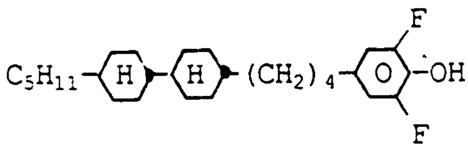

Die Umsetzung des Difluorbenzols mit BuLi und Borsäuretrimethylester zum Difluorphenol erfolgt analog Beispiel 1.

c) 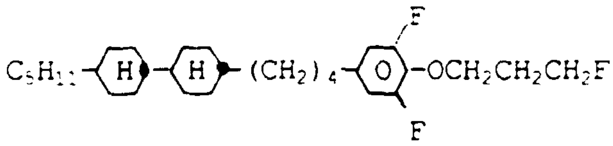

Analog Beispiel 3 c) wird das Difluorphenol aus Beispiel 7 b) mit 1-Chlor-3-fluorpropan zum Phenolether umgesetzt.

d) 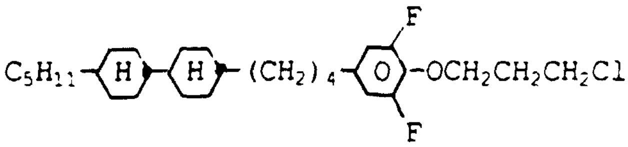

Analog Beispiel 3 d) wird das Produkt aus Beispiel 7 b) mit 1-Chlor-3-iod-propan umgesetzt.

Analog erhält man aus den entsprechenden Vorstufen der Formel II (L = H oder F) die folgenden erfindungsgemäßen Verbindungen der Formel

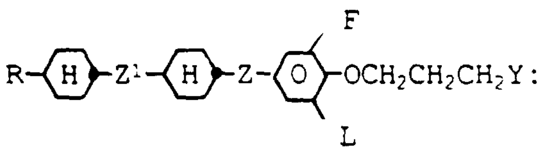

| R | 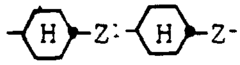 | L | Y |
|---|---|---|---|
| $CH_3$ | 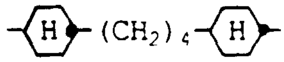 | H | F |
| $CH_3$ | 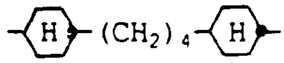 | H | Cl |
| $CH_3$ | 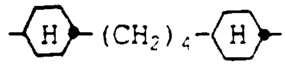 | F | Cl |
| $C_2H_5$ | 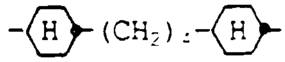 | H | F |
| $C_2H_5$ | 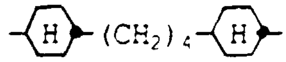 | F | F |

| R | 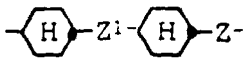 | L | Y |
|---|---|---|---|
| $n-C_3H_7$ | 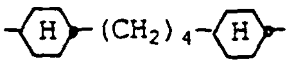 | H | F |
| $n-C_3H_7$ | 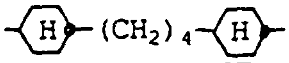 | H | Cl |
| $n-C_3H_7$ | 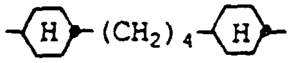 | F | Cl |
| $n-C_4H_9$ | 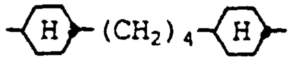 | F | F |
| $n-C_4H_9$ | 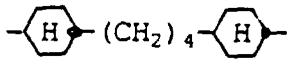 | F | Cl |
| $n-C_5H_{11}$ | 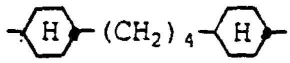 | H | F |
| $n-C_5H_{11}$ | 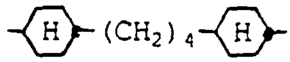 | F | F |
| $n-C_5H_{11}$ | 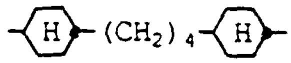 | F | Cl |
| $CH_3OCH_2$ | 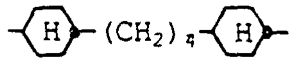 | H | F |
| $CH_3OCH_2$ | 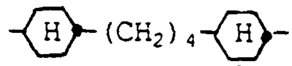 | F | F |
| $CH_2=CHCH_2CH_2$ | 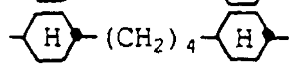 | H | F |
| $CH_2=CHCH_2CH_2$ | 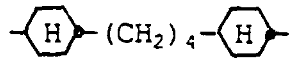 | F | F |
| $CH_2=CHCH_2CH_2$ | 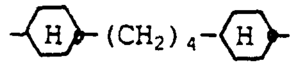 | F | Cl |

**Beispiel 7**

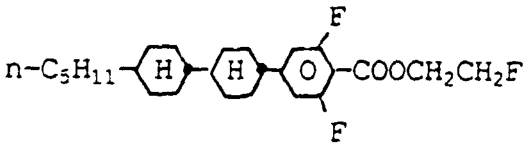

Zu einer Suspension von 26 mmol 4-trans-(4-Pentylcyclohexyl)-cyclohexyl-1-trans-3,5-difluorbenzoesäure (hergestellt nach Schema 7) in 160 ml Dichlormethan werden 26 mm 2-Fluorethanol und 0,1 g DMAP gegeben. Unter Rühren wird dann eine Lösung von 6,6 g DCC in 40 ml Dichlormethan zugetropft. Es wird über Nacht bei Raumtemperatur gerührt. Man versetzt mit Oxalsäure, rührt eine Stunde nach, chromatographiert mit Hexan und kristallisiert um. K 63 N 148,8 I

Analog erhält man die folgenden Verbindungen der Formel:

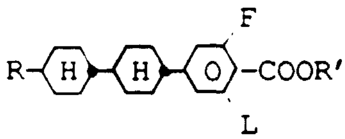

| R | L | R′ |
|---|---|---|
| $CH_3$ | H | $CH_2CH_2F$ |
| $C_2H_5$ | H | $CH_2CH_2F$ |
| $n-C_3H_7$ | H | $CH_2CH_2F$ |
| $n-C_4H_9$ | H | $CH_2CH_2F$ |
| $n-C_5H_{11}$ | H | $CH_2CH_2F$ |
| $CH_3OCH_2$ | H | $CH_2CH_2F$ |
| $CH_2=CHCH_2CH_2$ | H | $CH_2CH_2F$ |
| $CH_3$ | F | $CH_2CH_2F$ |
| $C_2H_5$ | F | $CH_2CH_2F$ |
| $n-C_3H_7$ | F | $CH_2CH_2F$ |
| $n-C_4H_9$ | F | $CH_2CH_2F$ |
| $CH_3OCH_2$ | F | $CH_2CH_2F$ |
| $CH_2=CHCH_2CH_2$ | F | $CH_2CH_2F$ |
| $CH_3$ | F | $CH_2CH_2Cl$ |
| $C_2H_5$ | F | $CH_2CH_2Cl$ |
| $n-C_3H_7$ | F | $CH_2CH_2Cl$ |
| $n-C_5H_{11}$ | F | $CH_2CH_2Cl$ |
| $CH_3OCH_2$ | F | $CH_2CH_2Cl$ |

| R | L | R' | |
|---|---|---|---|
| $CH_3$ | F | $CH_2CH_2CH_2Cl$ | |
| $C_2H_5$ | F | $CH_2CH_2CH_2Cl$ | |
| $n-C_3H_7$ | F | $CH_2CH_2CH_2Cl$ | |
| $n-C_5H_{11}$ | F | $CH_2CH_2CH_2Cl$ | K 58 N 102 I |
| $CH_3OCH_2$ | F | $CH_2CH_2CH_2Cl$ | |
| $CH_2=CHCH_2CH_2$ | F | $CH_2CH_2CH_2Cl$ | |
| $CH_3$ | F | $CH_2CH_2CH_2CF_3$ | |
| $C_2H_5$ | F | $CH_2CH_2CH_2CF_3$ | |
| $n-C_3H_7$ | F | $CH_2CH_2CH_2CF_3$ | |
| $n-C_5H_{11}$ | F | $CH_2CH_2CH_2CF_3$ | K 65 N 86,9 I |
| $CH_3OCH_2$ | F | $CH_2CH_2CH_2CF_3$ | |
| $CH_2=CHCH_2CH_2$ | F | $CH_2CH_2CH_2CF_3$ | |

**Beispiel 8**

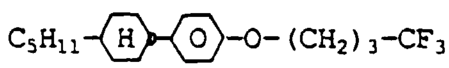

0,1 Mol 4-(4-Pentylcyclohexyl)-phenol, 0,102 Mol Triphenylphosphin und 0,1 Mol Trifluorbutan-4-ol werden in 250 ml Tetrahydrofuran bei Zimmertemperatur tropfenweise mit 0,102 Mol Diethylazodicarboxylat unter Kühlung versetzt. Anschließend wird über Nacht gerührt. Nach dem Eindampfen wird der Rückstand über Kieselgel mit Toluol filtriert.

Analog werden die folgenden Trifluoralkylether der Formel

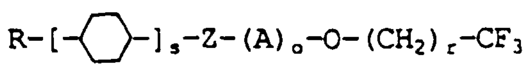

hergestellt:

| R | -[-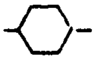-]$_s$-Z-(A)$_o$- | -O-$(CH_2)_r$-$CF_3$ |
|---|---|---|
| n-$C_3H_7$ | 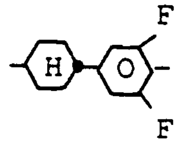 | $OCH_2CH_2CF_3$ |
| n-$C_5H_{11}$ | 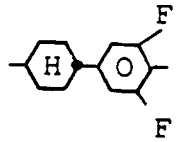 | $CCH_2CH_2CF_3$ |
| n-$C_3H_7$ | 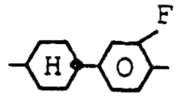 | $CCH_2CH_2CH_2CF_3$ |
| n-$C_5H_{11}$ | 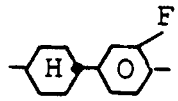 | $OCH_2CH_2CH_2CF_3$ |

| R | -[-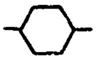-]$_s$-Z-(A)$_o$- | -O-$(CH_2)_r$-$CF_3$ |
|---|---|---|
| n-$C_3H_7$ | 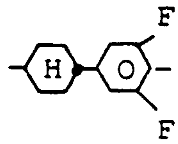 | $CCH_2CH_2CH_2CH_2CF_3$ |
| n-$C_5H_{11}$ | 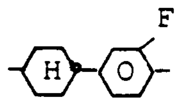 | $OCH_2CH_2CH_2CH_2CF_3$ |
| n-$C_3H_7$ | 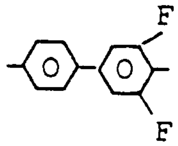 | $CCH_2CF_3$ |
| n-$C_5H_{11}$ | 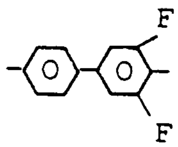 | $CCH_2CF_3$ |

| R | 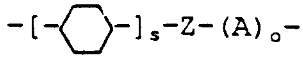 | $-O-(CH_2)_r-CF_3$ |
|---|---|---|
| $n-C_3H_7$ | 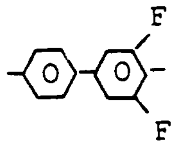 | $CCH_2CH_2CF_3$ |
| $n-C_5H_{11}$ | 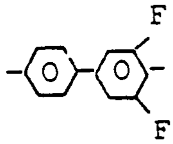 | $CCH_2CH_2CF_3$ |
| $n-C_3H_7$ | 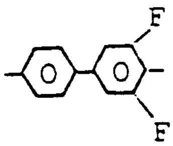 | $CCH_2CH_2CH_2CF_3$ |
| $n-C_5H_{11}$ | 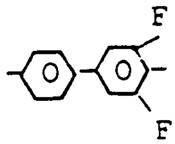 | $CCH_2CH_2CH_2CF_3$ |

| R | 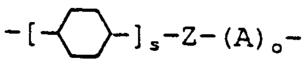 | $-O-(CH_2)_r-CF_3$ |
|---|---|---|
| $n-C_3H_7$ | 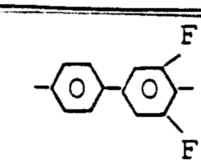 | $OCH_2CH_2CH_2CH_2CF_3$ |
| $n-C_5H_{11}$ | 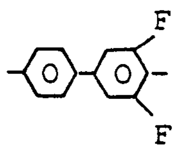 | $OCH_2CH_2CH_2CH_2CF_3$ |

| R | -[-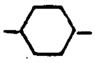-]$_s$-Z-(A)$_o$- | $-O-(CH_2)_r-CF_3$ |
|---|---|---|
| $n-C_3H_7$ | 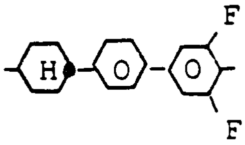 | $OCH_2CH_2CF_3$ |
| $CH_2=CHCH_2CH_2$ | 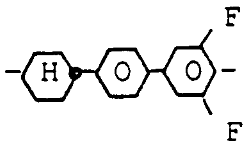 | $OCH_2CH_2CF_3$ |
| $n-C_3H_7$ | 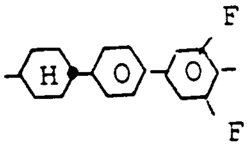 | $OCH_2CH_2CH_2CF_3$ |
| $n-C_5H_{11}$ | 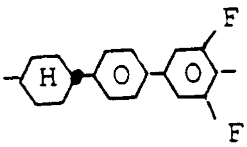 | $OCH_2CH_2CH_2CF_3$ |

| R | -[-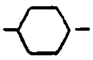-]$_s$-Z-(A)$_o$- | $-O-(CH_2)_r-CF_3$ |
|---|---|---|
| $n-C_3H_7$ | 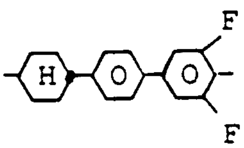 | $OCH_2CH_2CH_2CH_2CF_3$ |
| $n-C_3H_7$ | 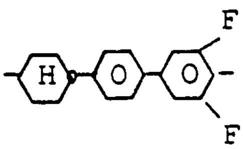 | $OCH_2CH_2CH_2CH_2CF_3$ |
| $n-C_5H_{11}$ | 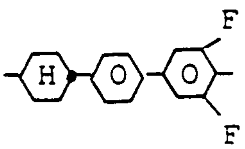 | $OCH_2CH_2CH_2CH_2CF_3$ |
| $n-C_3H_7$ | 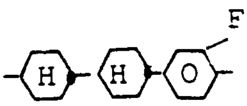 | $OCH_2CF_3$ |
| $n-C_5H_{11}$ | 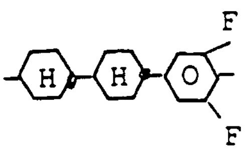 | $OCH_2CF_3$ |

| R | -[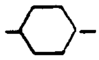-]$_s$-Z-(A)$_o$- | $-O-(CH_2)_r-CF_3$ |
|---|---|---|
| $n-C_3H_7$ | 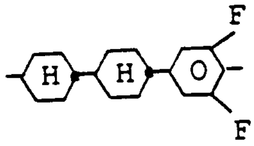 | $OCH_2CH_2CF_3$ |
| $n-C_5H_{11}$ | 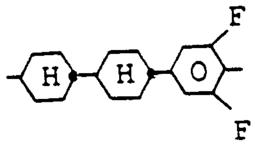 | $OCH_2CH_2CF_3$ |
| $n-C_3H_7$ | 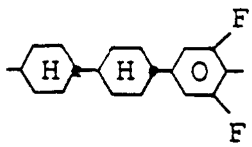 | $OCH_2CH_2CH_2CF_3$ |
| $n-C_5H_{11}$ | 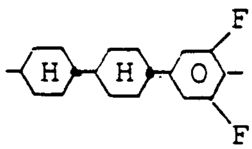 | $OCH_2CH_2CH_2CF_3$<br>K 61 $S_B$(56) N 145,2 I,<br>$\Delta n$ = 0,096 |
| $CH_3OCH_2$ | 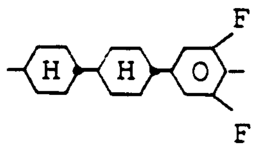 | $OCH_2CH_2CH_2CF_3$ |

| R | 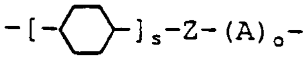 | $-O-(CH_2)_r-CF_3$ |
|---|---|---|
| $n-C_3H_7$ | 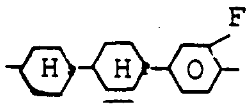 | $OCH_2CH_2CH_2CH_2CF_3$ |
| $C_2H_5$ | 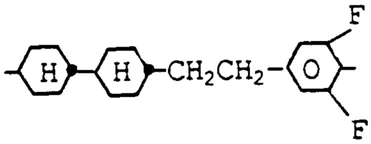 | $OCH_2CF_3$ |
| $n-C_3H_7$ | 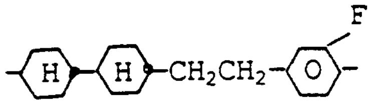 | $CCH_2CF_3$ |
| $n-C_3H_7$ | 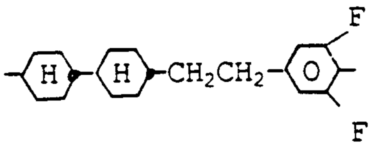 | $CCH_2CF_3$ |

| R | 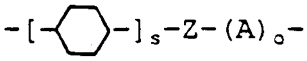 | $-O-(CH_2)_r-CF_3$ |
|---|---|---|
| $n-C_5H_{11}$ | 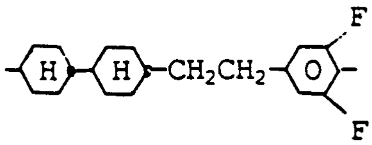 | $OCH_2CH_2CF_3$ |
| $CH_3OCH_2CH_2$ | 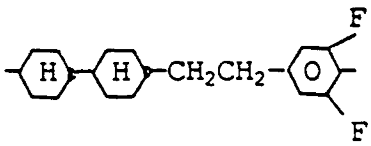 | $OCH_2CH_2CF_3$ |
| $CH_2 = CHCH_2CH_2$ | 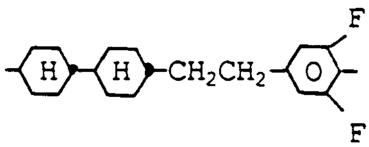 | $O-CH_2CH_2CH_2CF_3$ |
| $n-C_3H_7$ | 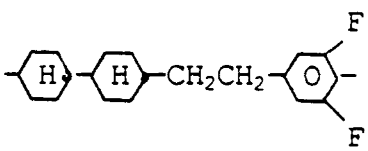 | $C-CH_2CH_2CH_2CF_3$ |

| R | 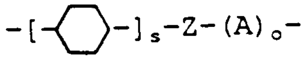 | $-O-(CH_2)_r-CF_3$ |
|---|---|---|
| $n-C_3H_7$ | 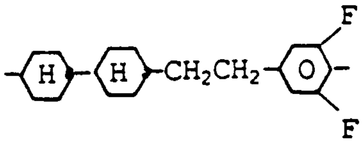 | $OCH_2CH_2CH_2CH_2CF_3$ |
| $n-C_5H_{11}$ | 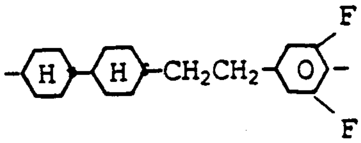 | $OCH_2CH_2CH_2CH_2CF_3$ |
| $n-C_5H_{11}$ | 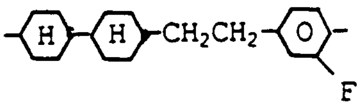 | $OCH_2CH_2CH_2CH_2CF_3$ |
| $n-C_3H_7$ | 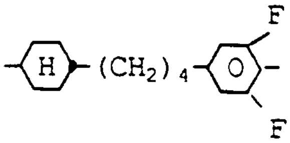 | $OCH_2CF_3$ |
| $n-C_5H_{11}$ | 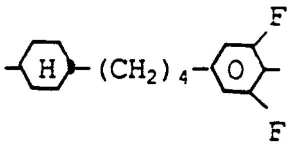 | $OCH_2CF_3$ |
| $CH_3OCH_2CH_2$ | 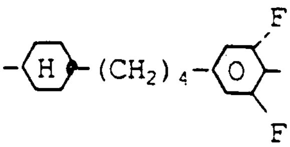 | $OCH_2CH_2CF_3$ |

| R | 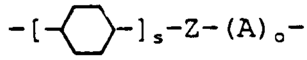 | $-O-(CH_2)_r-CF_3$ |
|---|---|---|
| $n-C_3H_7$ | 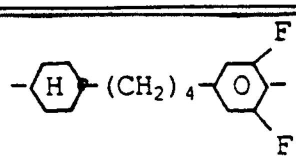 | $OCH_2CH_2CH_2CF_3$ |
| $n-C_5H_{11}$ | 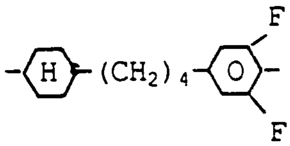 | $OCH_2CH_2CH_2CF_3$ |
| $C_2H_5$ | 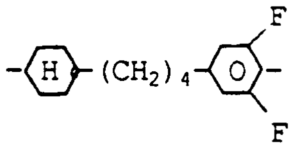 | $CCH_2CH_2CH_2CH_2CF_3$ |
| $n-C_3H_7$ | 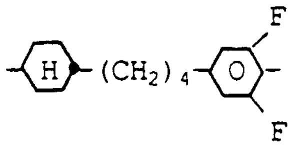 | $CCH_2CH_2CH_2CH_2CF_3$ |
| $n-C_5H_{11}$ | 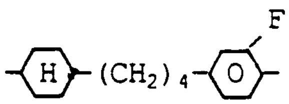 | $CCH_2CH_2CH_2CH_2CF_3$ |

**Beispiel 9**

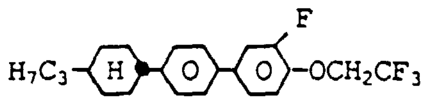

a) 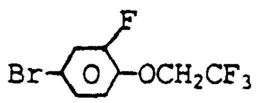

0,085 mol 4-Brom-2-fluorphenolat werden in 100 ml 1,3-Dimethyl-2-imidazolidinon gelöst, auf 140 °C erhitzt und tropfenweise mit 0,09 ml 2,2,2-Trifluorethylmethylsulfonat versetzt. Die Lösung wird 24 h bei 140 °C gerührt. Anschließend wird mit 500 ml Eiswasser versetzt und wie üblich aufgearbeitet.

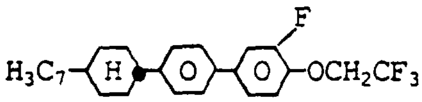

b)

0,015 mol 4-Brom-2-fluorphenol-(2,2,2-trifluorethyl)ether, werden mit 0,05 ml trans-n-Propyl-cyclohexylphenylboronsäure, 20 ml Toluol, 10 ml Ethanol, 0,030 mol Natriumcarbonat und 0,86 mmol Tetrakis(triphenylphosphin)-palladium (O) versetzt und 2 h unter Rückfluß gekocht. Nach Zugabe von 100 ml Petrolether (40-80°) wird wie üblich aufgearbeitet. K 81 $S_B$ 101 $S_A$ 133 N 139,6 I, Δn = 0,152

Analog werden die folgenden Verbindungen der Formel

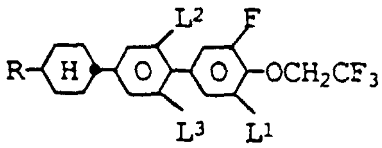

hergestellt:

| R | $L^1$ | $L^2$ | $L^3$ | |
|---|---|---|---|---|
| $C_2H_5$ | H | H | H | |
| $C_2H_5$ | F | H | H | |
| $C_2H_5$ | F | F | F | K 96 I, Δn = 0,105 |
| n-$C_3H_7$ | F | H | H | K 83 N 104,6 I, Δn = 0,152 |
| n-$C_3H_7$ | F | F | F | K 98 I, Δn = 0,111 |
| n-$C_5H_{11}$ | H | H | H | |
| n-$C_5H_{11}$ | F | H | H | |
| n-$C_5H_{11}$ | F | F | F | |
| $CH_3OCH_2$ | H | H | H | |
| $CH_3OCH_2$ | F | H | H | |
| $CH_3OCH_2$ | F | F | F | |
| $CH_2$=$CHCH_2CH_2$ | F | F | F | |

**Beispiel 15**

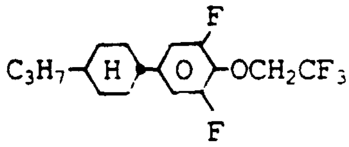

a) 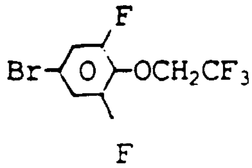

Analog Beispiel 14 a) werden 0,085 mol 4-Brom-2,6-difluorphenolat tropfenweise bei 140 °C mit 2,2,2-Trifluorethylmethylsulfonat versetzt.

b) 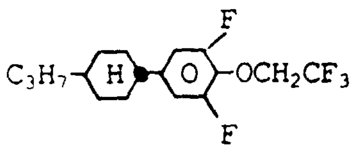

0,01 mol trans-4-n-Propylcyclohexylbromid werden in 15 ml eines Lösungsmittelgemisches von THF/Toluol (1:4) vorgelegt und mit 1,2 g Zinkchlorid und 0,14 g Lithiumgranulat versetzt. Das Gemisch wird unter Schutzgas und Rühren 4 Stunden zwischen 0 und 10 °C mit Ultraschall behandelt. Die erhaltene zinkorganische Verbindung wird mit 0,01 mol 4-Brom-2,6-difluorphenol-(2,2,2-trifluorethylether) und 2 mol-% 1,1'-Bis-(diphenylphosphino)-ferrocen-palladium(II)dichlorid versetzt und nach Entfernung des Ultraschallbades und der Kühlung 24 h bei Raumtemperatur gerührt. Es wird mit 10 ml gesättigter Ammoniumchlorid-Lösung versetzt, die organische Phase abgetrennt und wie üblich aufgearbeitet. K 5 I, $\Delta n = 0{,}038$

Analog werden die folgenden Verbindungen der Formel

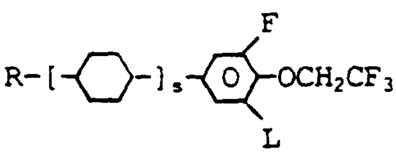

hergestellt:

| R | s | L | |
|---|---|---|---|
| n-$C_2H_5$ | 1 | H | |
| n-$C_2H_5$ | 1 | F | |
| n-$C_2H_5$ | 2 | H | K 65 $S_B$ 91 N 149,9 I, $\Delta n = 0{,}093$ |

(fortgesetzt)

| R | s | L | |
|---|---|---|---|
| $n-C_2H_5$ | 2 | F | K 65 N 100,2 I, $\Delta n$ = 0,059 |
| $n-C_3H_7$ | 1 | H | |
| $n-C_3H_7$ | 1 | F | |
| $n-C_3H_7$ | 2 | H | |
| $n-C_3H_7$ | 2 | F | K 69 N 140 I, $\Delta n$ = 0,092 |
| $n-C_5H_{11}$ | 1 | H | |
| $n-C_5H_{11}$ | 1 | F | |
| $n-C_5H_{11}$ | 2 | H | K 62 $S_B$ 92 N 151,7 I, $\Delta n$ = 0,097 |
| $n-C_5H_{11}$ | 2 | F | |
| $CH_3OCH_2$ | 1 | H | |
| $CH_3OCH_2$ | 1 | F | |
| $CH_3OCH_2$ | 2 | H | |
| $CH_3OCH_2$ | 2 | F | |
| $CH_2=CHCH_2CH_2$ | 1 | H | |
| $CH_2=CHCH_2CH_2$ | 1 | F | |
| $CH_2=CHCH_2CH_2$ | 2 | H | |
| $CH_2=CHCH_2CH_2$ | 2 | F | |

## Patentansprüche

**1.** Flüssigkristalline Verbindungen der Formel I,

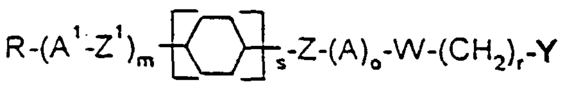

I

worin

R H, einen unsubstituierten, einen einfach durch CN oder $CF_3$ oder einen mindestens einfach durch Halogen substituierten Alkyl- oder Alkenylrest mit 1 bis 15 C-Atomen, wobei in diesen Resten auch eine oder mehrere $CH_2$-Gruppen jeweils unabhängig voneinander durch -O-, -S-,

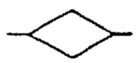,

-CO-, -CO-O-, -O-CO- oder -O-CO-O- so ersetzt sein können, daß O-Atome nicht direkt miteinander verknüpft sind,

$Z^1$ und Z jeweils unabhängig voneinander $-CH_2CH_2-$, CH=CH-, -C≡C- oder eine Einfachbindung, einer der Reste $Z^1$ und Z auch $-(CH_2)_4-$ oder $-CH=CH-CH_2CH_2-$,

A einen substituierten 1,4-Phenylenring, der in 2-, 2,3- oder 2,6-Stellung durch Fluor substituiert ist,

und $A^1$ ein trans-1,4-Cyclohexylen, worin auch ein oder zwei nicht benachbarte $CH_2$-Gruppen durch -O- ersetzt sein können, oder unsubstituiertes oder ein- oder zweifach durch Fluor und/oder Cl-Atome substituiertes 1,4-Phenylen, worin auch eine oder zwei CH-Gruppen durch N ersetzt sein können,

m 0, 1, 2 oder 3,

o 1 oder 2,

s 0, 1 oder 2, wobei $(m + s + o) \geq 2$,

W -O- oder -COO-,

r 1 bis 7, und

Y F, Cl oder $CF_3$ bedeuten.

**2.** Verbindungen der Formel I",

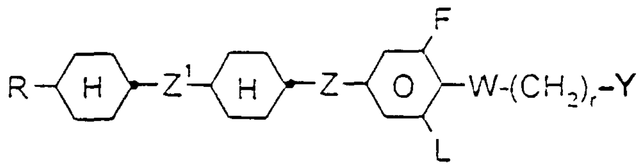

I"

worin

L H oder F bedeutet und R, $Z^1$, Z, W, Y und r die in Anspruch 1 angegebene Bedeutung besitzen.

**3.** Verbindungen der Formel Ic,

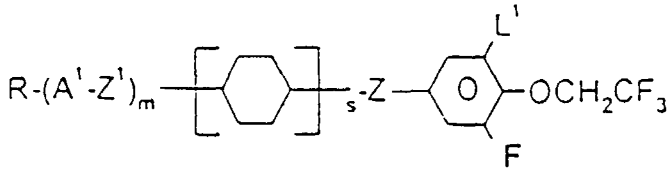

Ic

worin

$L^1$ H oder F bedeutet und R, $A^1$, $Z^1$, Z, m und s die in Anspruch 1 angegebene Bedeutung besitzen.

**4.** Verbindungen der Formel I3,

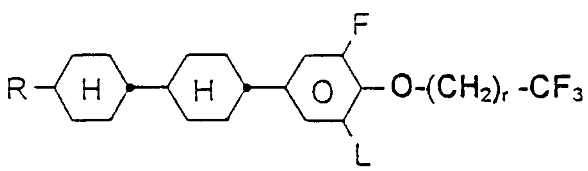

I3

worin

L H oder F,

bedeutet, und

R und r die in Anspruch 1 angegebene Bedeutung haben.

**5.** Verbindungen der Formel I48,

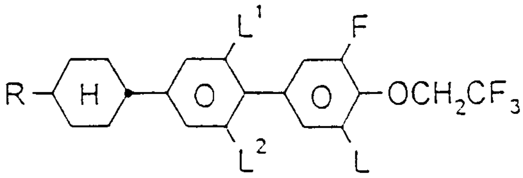

I48

worin

L$^1$, L$^2$ und L jeweils unabhängig voneinander H oder F

bedeuten, und

R die in Anspruch 1 angegebene Bedeutung hat.

**6.** Verbindungen der Formel I1,

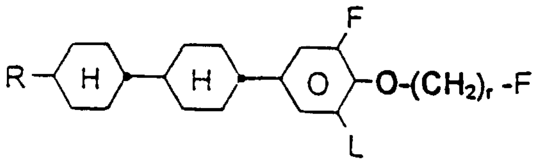

I1

worin

L H oder F bedeutet und
R und r die in Anspruch 1 angegebene Bedeutung haben.

**7.** Verbindungen der Formel

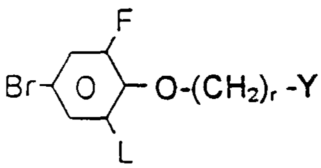

worin

L H oder F bedeutet und
r und Y die in Anspruch 1 angegebene Bedeutung haben.

**8.** Verwendung von Verbindungen der Formeln I als Komponenten flüssigkristalliner Medien.

**Claims**

**1.** Liquid-crystalline compounds of the formula I

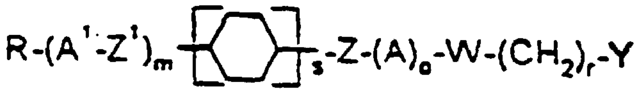

I

in which

R is H, an alkyl or alkenyl radical having 1 to 15 carbon atoms which is unsubstituted, monosubstituted by CN or $CF_3$ or at least mono-substituted by halogen, it also being possible for one or more $CH_2$ groups in these radicals each to be replaced, independently of one another, by -O-, -S-,

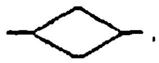

-CO-, -CO-O-, -O-CO- or -O-CO-O- in such a way that oxygen atoms are not linked directly to one another,

$Z^1$ and Z are each, independently of one another, $-CH_2CH_2-$, -CH=CH-, -C≡C- or a single bond, and one of the radicals $Z^1$ and Z is alternatively $-(CH_2)_4-$ or $-CH=CH-CH_2CH_2-$,

A is a substituted 1,4-phenylene ring which is substituted by fluorine in the 2-, 2,3- or 2,6-position,

and $A^1$ is a trans-1,4-cyclohexylene in which, in addition, one or two non-adjacent $CH_2$ groups may be replaced by -O-, or is 1,4-phenylene which is unsubstituted or monosubstituted or disubstituted by fluorine and/or Cl atoms and in which, in addition, one or two CH groups may be replaced by N,

m is 0, 1, 2 or 3,

o is 1 or 2,

s is 0, 1 or 2, where (m+s+o) is ≥ 2 ,

W is -O- or -COO-,

r is 1 to 7, and

Y is F, Cl or $CF_3$.

**2.** Compounds of the formula I''

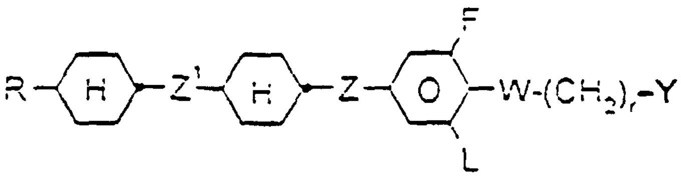

I''

in which

L is H or F, and R, $Z^1$, Z, W, Y and r are as defined in Claim 1.

**3.** Compounds of the formula Ic

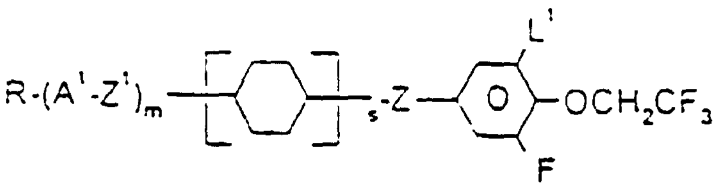

Ic

in which

$L^1$ is H or F, and R, $A^1$, $Z^1$, Z, m and s are as defined in Claim 1.

**4.** Compounds of the formula I3

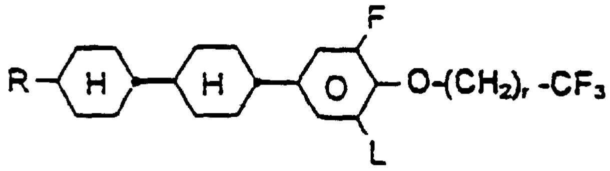

I3

in which

L is H or F, and R and r are as defined in Claim 1.

**5.** Compounds of the formula I48

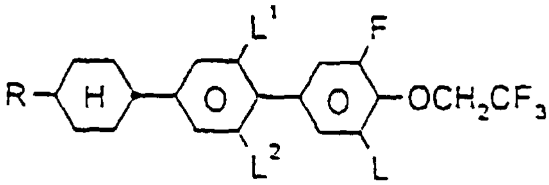

I48

in which

$L^2$, $L^2$ and L are each, independently of one another, H or F, and R is as defined in Claim 1.

**6.** Compounds of the formula I1

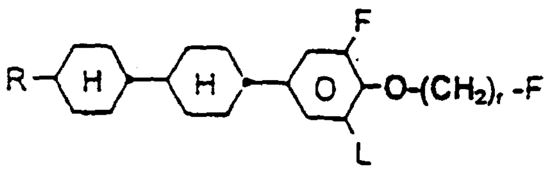

I1

in which

L is H or F and R and r are as defined in Claim 1.

**7.** Compounds of the formula

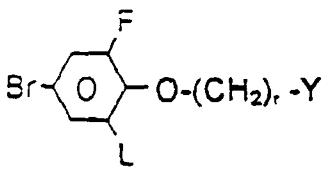

in which

L is H or F and
r and Y are as defined in claim 1.

**8.** Use of compounds of the formulae I as components of liquid-crystalline media.

## Revendications

**1.** Composés cristaux liquides de formule I

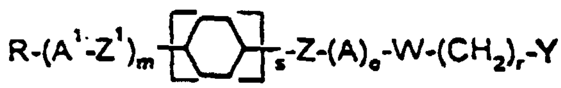

I

dans laquelle

R est H, un radical alkyle ou alcényle ayant de 1 à 15 atomes de carbone, non-substitué, une fois substitué par CN ou $CF_3$, ou au moins une fois substitué par des substituants halogéno, un ou plusieurs groupes $CH_2$ pouvant aussi, dans ces radicaux, être remplacé indépendamment les uns des autres par -O-, -S-,

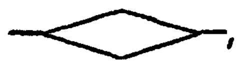

-CO-, -CO-O-, -O-CO- ou -O-CO-O-, de façon que les atomes d'oxygène ne soient pas directement liés les uns aux autres,
$Z^1$ et Z représentent chacun Indépendamment de l'autre $-CH_2CH_2-$, -CH=CH-, -C≡C- ou une liaison simple, l'un des radicaux $Z^1$ et Z étant aussi $-(CH_2)_4-$ ou $-CH=CH-CH_2-CH_2-$,
A est un noyau 1,4-phénylène substitué, qui est substitué par des substituants fluoro sur les positions 2-, 2,3- ou 2,6.
et $A^1$ est un radical trans-1,4-cyclohexylène, dans lequel aussi un ou deux groupes $CH_2$ non-voisins peuvent être remplacés par -O-, ou encore un groupe 1,4-phénylène non-substitué, ou une ou deux fois substitué par des atomes de fluor ou de chlore, où un ou deux des groupes CH peuvent aussi être remplacés par N,
m vaut 0, 1, 2 ou 3,
o vaut 1 ou 2,
s vaut 0, 1 ou 2, avec $(m + s + o) \geq 2$ ,
W est -O- ou -COO-,
r vaut de 1 à 7, et
Y est F, Cl ou $CF_3$.

**2.** Composés de formule I''

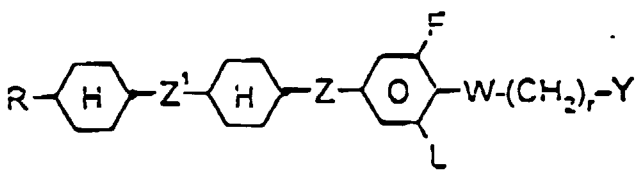

I''

dans laquelle

L est H ou F, et R, $Z^1$, Z, W, Y et r ont les significations données dans la revendication 1.

**3.** Composés de formule Ic

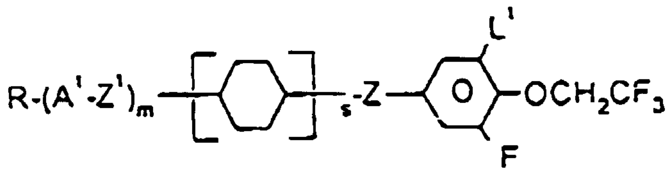

Ic

dans laquelle

$L^1$ est H ou F, et R, $A^1$, $Z^1$, Z, m et s ont les significations données dans la revendication 1.

**4.** Composés de formule I3

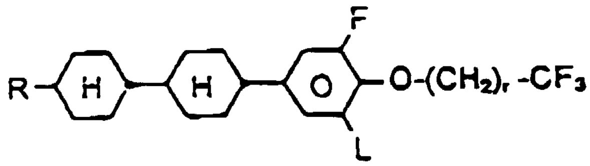

I3

dans laquelle

L est H ou F, et R et r ont les significations données dans la revendication 1.

**5.** Composés selon la formule I48

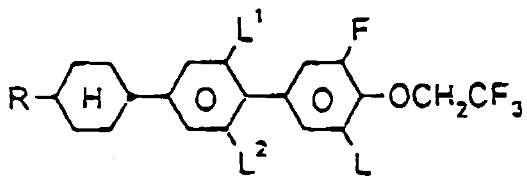

I48

dans laquelle

$L^1$, $L^2$ et L représentent chacun indépendamment des autres H ou F, et R a les significations données dans la revendication 1.

**6.** Composés de formule I1

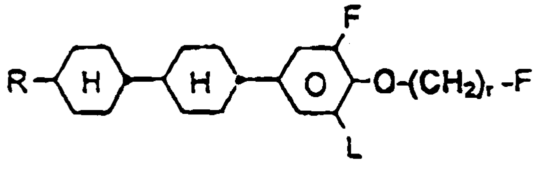

I1

dans laquelle

L est H ou F, et R et r ont les significations données dans la revendication 1.

**7.** Composés de formule

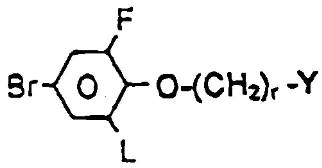

dans laquelle

L est H ou F, et r et Y ont les significations données dans la revendication 1.

**8.** Utilisation de composés ayant les formules I en tant que composants de milieux cristaux liquides.